# EUROPEAN PATENT APPLICATION

(11) **EP 4 353 279 A1**
(43) Date of publication of application: **17.04.2024**
(21) Application number: 22820330.3
(22) Date of filing: 09.06.2022
(51) Int. Cl.: A61M 5/158

(54) **NEEDLE ASSEMBLY**

(30) Priority: 10.06.2021 JP 2021097546
(71) Applicant: Nipro Corporation, Settsu-shi, Osaka 566-8510 (JP)
(72) Inventor: FUJIWARA, Yuta, Osaka-shi, Osaka 531-8510 (JP); YOSHIDA, Eimi, Osaka-shi, Osaka 531-8510 (JP); SHIMAMOTO, Jumpei, Osaka-shi, Osaka 531-8510 (JP); MATSUNAGA, Takuya, Osaka-shi, Osaka 531-8510 (JP)
(74) Representative: Slingsby Partners LLP
(86) International application number: PCT/JP2022/023378
(87) International publication number: WO 2022/260151

(57) **Abstract**

A needle assembly 10 including: a pair of variable plate-shaped parts 40a, 40b arranged in opposition and bendable at upper and lower portions 70, 68; a bendable bent part 46 provided in a middle in a height direction of each plate-shaped part 40; and a needle hub 24 of a puncture needle 12 held by the upper portions 70, 70 such that the plate-shaped parts 40a, 40b can extend and contract vertically, namely in a puncture direction. With the plate-shaped parts 40a, 40b contracted, a position of a needle tip 16 of the puncture needle 12 is a puncture position where puncture is possible, and with the plate-shaped parts 40a, 40b extended, a position of the needle tip 16 is a needle removal position where the needle is removed. The lower portions 68, 68 of the plate-shaped parts 40a, 40b are connected via a bottom plate 54 to be overlapped on a patient's skin A.

## Description

### TECHNICAL FIELD

This invention relates to a needle assembly including a puncture needle that punctures, for example, a subcutaneous implantable port or the like.

### BACKGROUND ART

Conventionally, a needle assembly has been known for puncturing a subcutaneous implantable port buried under the skin of, for example, the chest, the upper arm, etc. of a patient. The needle assembly includes a hollow puncture needle that punctures a septum (puncture portion formed of compressed silicone rubber or the like) of the subcutaneous implantable port.

Meanwhile, as a needle assembly, a structure that enables one-handed operation has also been proposed, for example, in PCT Japanese Translation Patent Publication No. JP-A-2002-529156 (Patent Document 1). According to the needle assembly that enables one-handed operation, for example, it is easy for a patient to remove a stuck needle assembly from a subcutaneous implantable port by oneself.

### BACKGROUND ART DOCUMENT

### PATENT DOCUMENT

Patent document 1: JP-A-2002-529156

### SUMMARY OF THE INVENTION

### PROBLEM THE INVENTION ATTEMPTS TO SOLVE

However, research conducted by the inventors revealed that the needle assembly with a hypodermic needle safety enclosure disclosed in Patent Document 1 still has some points to be improved regarding operability, safety, etc. during puncture, indwelling, and removal of a needle.

It is therefore one object of the present invention to provide a needle assembly of novel structure that improves the points to be improved regarding operability, safety, etc. during puncture, indwelling, and removal of a needle, which are inherent in conventional needle assemblies.

### MEANS FOR SOLVING THE PROBLEM

Hereinafter, preferred embodiments for grasping the present invention will be described. However, each preferred embodiment described below is exemplary and can be appropriately combined with each other. Besides, a plurality of elements described in each preferred embodiment can be recognized and adopted as independently as possible, or can also be appropriately combined with any element described in other preferred embodiments. By so doing, in the present invention, various other preferred embodiments can be realized without being limited to those described below.

A first preferred embodiment provides a needle assembly comprising: a pair of variable plate-shaped parts arranged in opposition to each other and being mutually bendable at both upper portions and lower portions, the pair of variable plate-shaped parts each including a bent part in a middle portion in a height direction, the bent part being allowed to undergo bending deformation; and a needle hub of a puncture needle held by the upper portions of the pair of variable plate-shaped parts such that the pair of variable plate-shaped parts are allowed to deform so as to extend and contract in a vertical direction that is a puncture direction of the puncture needle, wherein in a contracted state of the pair of variable plate-shaped parts, a position of a needle tip of the puncture needle is defined as a puncture position where puncture is possible, while in an extended state of the pair of variable plate-shaped parts, a position of the needle tip is defined as a needle removal position where the puncture needle is removed, and a lock mechanism configured to hold the pair of variable plate-shaped parts in the extended state is provided on opposed inside faces of the pair of variable plate-shaped parts.

According to the needle assembly structured following this preferred embodiment, after the puncture needle is removed, the pair of variable plate-shaped parts are held in the extended state by the lock mechanism, so that the needle tip of the puncture needle is held in the needle removal position to prevent accidental puncture of the needle tip. Additionally, since the lock mechanism is provided on the opposed inside faces of the variable plate-shaped parts, it is difficult to touch the lock mechanism.

A second preferred embodiment provides the needle assembly according to the first preferred embodiment, wherein the lock mechanism is provided above the bent parts of the variable plate-shaped parts.

According to the needle assembly structured following this preferred embodiment, the lock mechanism provided on the opposed inside faces of the pair of variable plate-shaped parts is located on the side away from the needle tip. This makes it easy to surely obtain a space for the lock mechanism even when, for example, a protective structure for the needle tip etc. is provided.

A third preferred embodiment provides a needle assembly comprising: a pair of variable plate-shaped parts arranged in opposition to each other and being mutually bendable at both upper portions and lower portions, the pair of variable plate-shaped parts each including a bent part in a middle portion in a height direction, the bent part being allowed to undergo bending deformation; and a needle hub of a puncture needle held by the upper portions of the pair of variable plate-shaped parts such that the pair of variable plate-shaped parts are allowed to deform so as to extend and contract in a vertical direction that is a puncture direction of the puncture needle, wherein in a contracted state of the pair of variable plate-shaped parts, a position of a needle tip of the puncture needle is defined as a puncture position where puncture is possible, while in an extended state of the pair of variable plate-shaped parts, a position of the needle tip is defined as a needle removal position where the puncture needle is removed, and the lower portions of the pair of variable plate-shaped parts are interconnected via a bottom plate that is configured to be overlapped on a skin of a patient.

According to the needle assembly structured following this preferred embodiment, the puncture needle can be pulled out by extension deformation of the pair of variable plate-shaped parts, and for example, the puncture needle can be removed by one-handed operation. In this way, since puncture and removal of the puncture needle can be easily operated with one hand, it is possible, for example, for the patient to remove the puncture needle from the subcutaneous implantable port by oneself.

The pair of variable plate-shaped parts are connected via the bottom plate, and the bottom plate is configured to be overlapped on the patient's skin. Accordingly, the needle assembly is in planar contact with the patient's skin by the bottom plate, and the contact area between the needle assembly and the patient's skin is largely obtained, thereby achieving reduction in contact pressure. This reduces pain and discomfort due to contact by the needle assembly during indwelling of the needle assembly in a puncture state. Besides, tilting of the needle assembly is suppressed during taping work at the time of indwelling, during needle removal, etc., thereby reducing pain, improving work efficiency, and the like.

Besides, the bottom plate is less deformed and displaced than the pair of variable plate-shaped parts when the variable plate-shaped parts extend and contract as well, making it easy to surely obtain stable contact with the patient's skin.

A fourth preferred embodiment provides the needle assembly according to the third preferred embodiment, wherein the bottom plate includes a projecting part projecting outward with respect to the variable plate-shaped parts in a length direction parallel to a bending axis of the variable plate-shaped parts.

According to the needle assembly structured following this preferred embodiment, the contact area between the bottom plate and the patient's skin can be made larger by the bottom plate including the projecting part. Accordingly, reduction in pain due to decreased contact pressure, efficiency in fixing work due to suppressed wobbling, etc. are more effectively achieved.

A fifth preferred embodiment provides the needle assembly according to the fourth preferred embodiment, wherein the bottom plate is widened at the projecting part.

According to the needle assembly structured following this preferred embodiment, the bottom plate is widened at the projecting part projecting outward with respect to the pair of variable plate-shaped parts. This makes it possible to obtain a larger area of the bottom plate while making it difficult for the bottom plate to affect the size etc. of the connected portion of the pair of variable plate-shaped parts. Besides, since the length of the contact surface between the bottom plate and the patient's skin is enlarged in the width direction of the projecting part, resistance to tilting of the needle assembly can be greatly obtained, thereby suppressing tilting of the needle assembly.

A sixth preferred embodiment provides the needle assembly according to the fourth or fifth preferred embodiment, wherein the projecting part is arranged in opposition to the needle hub, and the projecting part includes a support part projecting toward the needle hub.

According to the needle assembly structured following this preferred embodiment, in the contracted state of the pair of variable plate-shaped parts, the support part provided to the projecting part comes into contact with the needle hub, thereby restricting movements such as tilting of the needle hub. Therefore, shake of the needle tip etc. during puncture due to unnecessary movement of the needle hub is suppressed.

A seventh preferred embodiment provides a needle assembly comprising: a pair of variable plate-shaped parts arranged in opposition to each other and being mutually bendable at both upper portions and lower portions, the pair of variable plate-shaped parts each including a bent part in a middle portion in a height direction, the bent part being allowed to undergo bending deformation; and a needle hub of a puncture needle held by the upper portions of the pair of variable plate-shaped parts such that the pair of variable plate-shaped parts are allowed to deform so as to extend and contract in a vertical direction that is a puncture direction of the puncture needle, wherein in a contracted state of the pair of variable plate-shaped parts, a position of a needle tip of the puncture needle is defined as a puncture position where puncture is possible, while in an extended state of the pair of variable plate-shaped parts, a position of the needle tip is defined as a needle removal position where the puncture needle is removed, the needle hub includes a coupling part extending in a direction intersecting the puncture direction of the puncture needle, the puncture needle projects in the puncture direction from a lengthwise middle of the coupling part, and the upper portions of the pair of variable plate-shaped parts are attached to the coupling part on lengthwise opposite sides of a portion from which the puncture needle projects.

According to the needle assembly structured following this preferred embodiment, force due to extension and contraction of the pair of variable plate-shaped parts is input to the needle hub on the opposite sides of the projecting portion of the puncture needle from the needle hub. Accordingly, for example, when inducing extension deformation of the pair of variable plate-shaped parts and removing the needle, moment acting on the needle hub will be reduced, making it difficult for the needle hub to tilt, and the projecting portion of the puncture needle from the needle hub to be removed from the patient is unlikely to tilt due to the tilting of the needle hub. Therefore, smooth needle removal is possible, and pain caused by the tilting of the puncture needle in the puncture state is reduced.

An eighth preferred embodiment provides the needle assembly according to the seventh preferred embodiment, wherein the upper portions of the pair of variable plate-shaped parts comprise respective fitting parts, and the coupling part is rotatably fitted in the fitting parts of the pair of variable plate-shaped parts such that mutual bending of the pair of variable plate-shaped parts is permitted.

According to the needle assembly structured following this preferred embodiment, the needle hub and the upper portions of the pair of variable plate-shaped parts can be easily coupled by fitting between the coupling part and the pair of fitting parts. Additionally, the coupling part is fitted into the fitting parts in a state that permits bending of the variable plate-shaped parts at the bent part. Thus, extensional and contractive deformation of the variable plate-shaped parts is not hampered by the fitting between the coupling part and the fitting parts.

A ninth preferred embodiment provides a needle assembly comprising: a pair of variable plate-shaped parts arranged in opposition to each other and being mutually bendable at both upper portions and lower portions, the pair of variable plate-shaped parts each including a bent part in a middle portion in a height direction, the bent part being allowed to undergo bending deformation; and a needle hub of a puncture needle held by the upper portions of the pair of variable plate-shaped parts such that the pair of variable plate-shaped parts are allowed to deform so as to extend and contract in a vertical direction that is a puncture direction of the puncture needle, wherein in a contracted state of the pair of variable plate-shaped parts, a position of a needle tip of the puncture needle is defined as a puncture position where puncture is possible, while in an extended state of the pair of variable plate-shaped parts, a position of the needle tip is defined as a needle removal position where the puncture needle is removed, a grip piece configured to be gripped during puncture of the puncture needle is provided, and the grip piece is located between opposite ends of the variable plate-shaped parts in a length direction parallel to a bending axis of the variable plate-shaped parts.

According to the needle assembly structured following this preferred embodiment, when gripping the grip piece to puncture, unnecessary moment is unlikely to act on the puncture needle by the force being exerted on the grip piece in the puncture direction. Thus, the puncture needle is unlikely to tilt, thereby realizing smooth puncture. In particular, if the grip piece and the needle tip of the puncture needle are arranged side by side in the puncture direction, transmission efficiency of the force in the puncture direction from the grip piece to the puncture needle is increased, thereby realizing smoother puncture.

A tenth preferred embodiment provides the needle assembly according to the ninth preferred embodiment, wherein the grip piece extends from at least one of the variable plate-shaped parts, and a movable part is provided, the movable part permitting bending of the grip piece with respect to the at least one of the variable plate-shaped parts.

According to the needle assembly structured following this preferred embodiment, the grip piece extends from the variable plate-shaped part, which makes it easy to provide a movable part at the connected portion between the grip piece and the variable plate-shaped part. Besides, during puncture, the movable part separates the grip piece from the variable plate-shaped part, so that the user can easily grip the grip piece by pinching it with the fingertips. Furthermore, for example, when the needle assembly is indwelled after puncture, by the grip piece being brought closer to the variable plate-shaped part by the movable part, projection of the grip piece can be suppressed, thereby facilitating fixing by taping etc.

An eleventh preferred embodiment provides a needle assembly comprising: a pair of variable plate-shaped parts arranged in opposition to each other and being mutually bendable at both upper portions and lower portions, the pair of variable plate-shaped parts each including a bent part in a middle portion in a height direction, the bent part being allowed to undergo bending deformation; and a needle hub of a puncture needle held by the upper portions of the pair of variable plate-shaped parts such that the pair of variable plate-shaped parts are allowed to deform so as to extend and contract in a vertical direction that is a puncture direction of the puncture needle, wherein in a contracted state of the pair of variable plate-shaped parts, a position of a needle tip of the puncture needle is defined as a puncture position where puncture is possible, while in an extended state of the pair of variable plate-shaped parts, a position of the needle tip is defined as a needle removal position where the puncture needle is removed, and a protective part is provided, the protective part being configured to prevent the needle tip of the puncture needle from projecting to an outside in the extended state of the pair of variable plate-shaped parts.

According to the needle assembly structured following this preferred embodiment, in the extended state of the pair of variable plate-shaped parts, the needle tip of the puncture needle can be prevented from projecting to the outside from between the variable plate-shaped parts. This makes it difficult to accidentally touch the needle tip, whereby accidental puncture etc. are unlikely to become a problem.

A twelfth preferred embodiment provides a needle assembly comprising: a pair of variable plate-shaped parts arranged in opposition to each other and being mutually bendable at both upper portions and lower portions, the pair of variable plate-shaped parts each including a bent part in a middle portion in a height direction, the bent part being allowed to undergo bending deformation; and a needle hub of a puncture needle held by the upper portions of the pair of variable plate-shaped parts such that the pair of variable plate-shaped parts are allowed to deform so as to extend and contract in a vertical direction that is a puncture direction of the puncture needle, wherein in a contracted state of the pair of variable plate-shaped parts, a position of a needle tip of the puncture needle is defined as a puncture position where puncture is possible, while in an extended state of the pair of variable plate-shaped parts, a position of the needle tip is defined as a needle removal position where the puncture needle is removed, and the bent part of each variable plate-shaped part is defined as an input bent part configured to be pressed when the variable plate-shaped part undergoes extension deformation, and the variable plate-shaped part is thickened toward the input bent part.

According to the needle assembly structured following this preferred embodiment, the variable plate-shaped part is thickened toward the input bent part, thereby increasing the contact area of the input bent part with the fingers etc. Therefore, the contact area of the variable plate-shaped part with the fingers, etc. can be increased. Therefore, force can be stably applied to the input bent part, and pain caused by the input bent part biting into the fingers etc. can be reduced when the force is applied.

A thirteenth preferred embodiment provides a needle assembly comprising: a pair of variable plate-shaped parts arranged in opposition to each other and being mutually bendable at both upper portions and lower portions, the pair of variable plate-shaped parts each including at least one bent part in a middle portion in a height direction, the at least one bent part being allowed to undergo bending deformation; and a needle hub of a puncture needle held by the upper portions of the pair of variable plate-shaped parts such that the pair of variable plate-shaped parts are allowed to deform so as to extend and contract in a vertical direction that is a puncture direction of the puncture needle, wherein in a contracted state of the pair of variable plate-shaped parts, a position of a needle tip of the puncture needle is defined as a puncture position where puncture is possible, while in an extended state of the pair of variable plate-shaped parts, a position of the needle tip is defined as a needle removal position where the puncture needle is removed, and the at least one bent part of each variable plate-shaped part comprises a plurality of bent parts, and a portion between two of the bent parts adjacent to each other in the height direction is defined as an input plate configured to be pressed when the variable plate-shaped part undergoes extension deformation.

According to the needle assembly structured following this preferred embodiment, the portion to which force is applied by the fingers etc. when inducing extension deformation of the pair of variable plate-shaped parts is defined as the plate-shaped input plate, thereby increasing the contact area with the fingers etc. Therefore, force can be stably applied to the input plate, and pain caused by the input plate biting into the fingers etc. can be reduced when the force is applied.

A fourteenth preferred embodiment provides a needle assembly comprising: a pair of variable plate-shaped parts arranged in opposition to each other and being mutually bendable at both upper portions and lower portions, the pair of variable plate-shaped parts each including a bent part in a middle portion in a height direction, the bent part being allowed to undergo bending deformation; and a needle hub of a puncture needle held by the upper portions of the pair of variable plate-shaped parts such that the pair of variable plate-shaped parts are allowed to deform so as to extend and contract in a vertical direction that is a puncture direction of the puncture needle, wherein in a contracted state of the pair of variable plate-shaped parts, a position of a needle tip of the puncture needle is defined as a puncture position where puncture is possible, while in an extended state of the pair of variable plate-shaped parts, a position of the needle tip is defined as a needle removal position where the puncture needle is removed, and a fixing piece configured to be overlapped on and fixed to a skin of a patient projects outward with respect to the pair of variable plate-shaped parts in a length direction parallel to a bending axis of the variable plate-shaped parts.

According to the needle assembly structured following this preferred embodiment, by fixing the fixing piece to the patient's skin with a medical tape etc., the needle assembly will be indwelled in a more stably fixed manner to the patient's body surface than, for example, when it is fixed with the medical tape etc. to cover the pair of variable plate-shaped parts only. Besides, since the fixing piece projects outward with respect to the variable plate-shaped parts in the width direction, the variable plate-shaped parts are unlikely to be obstacles when fixing the fixing piece with the medical tape etc.

A fifteenth preferred embodiment provides the needle assembly according to any one of the first to fourteenth preferred embodiments, wherein an elastically deformable buffer body is provided on a surface configured to be overlapped in contact with a skin of a patient.

According to the needle assembly structured according to this preferred embodiment, the buffer body is arranged between the needle assembly and the patient's skin, thereby reducing pain and discomfort caused by touch of the needle assembly.

A sixteenth preferred embodiment provides the needle assembly according to any one of the first to fifteenth preferred embodiments, wherein the needle tip of the puncture needle is configured to be housed between the pair of variable plate-shaped parts in the extended state of the pair of variable plate-shaped parts.

According to the needle assembly structured following this preferred embodiment, by inducing extension deformation of the pair of variable plate-shaped parts and pulling out the puncture needle, the needle tip of the puncture needle is housed between the variable plate-shaped parts. This prevents the needle tip of the removed puncture needle from being exposed, thereby preventing accidental puncture etc.

A seventeenth preferred embodiment provides a needle assembly comprising: a pair of variable plate-shaped parts arranged in opposition to each other and being mutually bendable at both upper portions and lower portions, the pair of variable plate-shaped parts each including a bent part in a middle portion in a height direction, the bent part being allowed to undergo bending deformation; and a needle hub of a puncture needle held by the upper portions of the pair of variable plate-shaped parts such that the pair of variable plate-shaped parts are allowed to deform so as to extend and contract in a vertical direction that is a puncture direction of the puncture needle, wherein in a contracted state of the pair of variable plate-shaped parts, a position of a needle tip of the puncture needle is defined as a puncture position where puncture is possible, while in an extended state of the pair of variable plate-shaped parts, a position of the needle tip is defined as a needle removal position where the puncture needle is removed, a grip piece configured to be gripped during puncture of the puncture needle is provided, and the puncture needle extends in the puncture direction at a position overlapping with the grip piece in a bending axis direction of the variable plate-shaped parts.

According to the needle assembly structured following this preferred embodiment, when, for example, the variable plate-shaped part is extended or contracted by input to the grip piece to move the needle tip of the puncture needle to the puncture position or the needle removal position etc., the force from the grip piece is transmitted to the distal part of the puncture needle having the needle tip in a balanced manner with the grip piece gripped with the fingers. This enables smooth puncture and needle removal without tilting of the puncture needle etc.

An eighteenth preferred embodiment provides a needle assembly comprising: a pair of variable plate-shaped parts arranged in opposition to each other and being mutually bendable at both upper portions and lower portions, the pair of variable plate-shaped parts each including a bent part in a middle portion in a height direction, the bent part being allowed to undergo bending deformation; and a needle hub of a puncture needle held by the upper portions of the pair of variable plate-shaped parts such that the pair of variable plate-shaped parts are allowed to deform so as to extend and contract in a vertical direction that is a puncture direction of the puncture needle, wherein in a contracted state of the pair of variable plate-shaped parts, a position of a needle tip of the puncture needle is defined as a puncture position where puncture is possible, while in an extended state of the pair of variable plate-shaped parts, a position of the needle tip is defined as a needle removal position where the puncture needle is removed, the lower portions of the pair of variable plate-shaped parts are interconnected via a bottom plate that is configured to be overlapped on a skin of a patient, and a position regulating part is provided, the position regulating part being configured to limit relative displacement between the needle hub and the bottom plate when the needle tip is in the puncture position.

According to the needle assembly structured following this preferred embodiment, for example, the position regulating part limits the relative displacement between the needle hub and the bottom plate in the direction of opposition of the pair of variable plate-shaped parts, thereby suppressing shake of the needle tip of the puncture needle. Also, for example, by limiting the relative displacement between the needle hub and the bottom plate in the direction of mutual separation, the needle tip of the puncture needle can be held in the puncture position during puncture, thereby preventing accidental removal of the puncture needle (release of the puncture state) and the like.

A nineteenth preferred embodiment provides the needle assembly according to the eighteenth preferred embodiment, wherein the bottom plate includes a needle passage hole through which the puncture needle is configured to be inserted when the needle tip of the puncture needle is in the puncture position, and an external-fit part projecting toward the needle hub from around the needle passage hole, the needle hub includes an insertion part extending toward the bottom plate, and the position regulating part is constituted by the insertion part being inserted into the external-fit part, the position regulating part limiting the relative displacement between the needle hub and the bottom plate in at least one of a direction of separation of the needle hub and the bottom plate and a direction of opposition of the pair of variable plate-shaped parts.

According to the needle assembly structured following this preferred embodiment, even if the needle passage hole is sufficiently large in diameter for the puncture needle, the relative displacement between the needle hub and the bottom plate can be limited in the direction of mutual separation and in the direction of opposition of the pair of variable plate-shaped parts by the insertion part of the needle hub being inserted into the external-fit part of the bottom plate when the needle tip is in the puncture position.

A twentieth preferred embodiment provides a needle assembly comprising a pair of variable plate-shaped parts arranged in opposition to each other and being mutually bendable at both upper portions and lower portions, the pair of variable plate-shaped parts each including a bent part in a middle portion in a height direction, the bent part being allowed to undergo bending deformation, wherein the upper portions of the pair of variable plate-shaped parts comprise respective fitting parts of groove shape attached to an outer circumferential surface of a needle hub of a puncture needle, the fitting parts being slidable with respect to the needle hub in a circumferential direction of the needle hub such that the pair of variable plate-shaped parts are allowed to deform so as to extend and contract in a vertical direction that is a puncture direction of the puncture needle, in a contracted state of the pair of variable plate-shaped parts, a position of a needle tip of the puncture needle is defined as a puncture position where puncture is possible, while in an extended state of the pair of variable plate-shaped parts, a position of the needle tip is defined as a needle removal position where the puncture needle is removed, and a stopper protrusion is provided on an outer circumferential surface of the fitting part of at least one of the variable plate-shaped parts, the stopper protrusion being configured to be engaged to the other of the variable plate-shaped parts to prevent the needle tip from moving from the needle removal position to the puncture position.

According to the needle assembly structured following this preferred embodiment, sliding of the fitting part with respect to the needle hub is prevented by the stopper protrusion being engaged with the other variable plate-shaped part, thereby preventing shift of the pair of variable plate-shaped parts from the extended state to the contracted state. This makes it possible to prevent the puncture needle from being re-exposed after needle removal and becoming available for puncture.

### EFFECT OF THE INVENTION

According to the present invention, it is possible to provide an improved needle assembly while reliably obtaining one-handed operability during puncture and removal of the puncture needle.

### BRIEF DESCRIPTION OF THE DRAWINGS

FIG. 1 is a perspective view of a needle assembly as a first practical embodiment of the present invention, showing a contracted state where a needle tip of a puncture needle is in a puncture position.
FIG. 2 is a front view of the needle assembly shown in FIG. 1.
FIG. 3 is a right side view of the needle assembly shown in FIG. 1.
FIG. 4 is a bottom plan view of the needle assembly shown in FIG. 1.
FIG. 5 is a cross sectional view taken along line 5-5 of FIG. 2.
FIG. 6 is a perspective view of a wing member constituting the needle assembly shown in FIG. 1.
FIG. 7 is a front view of the wing member shown in FIG. 6.
FIG. 8 is a front view showing an embodiment of the needle assembly shown in FIG. 1 during puncture.
FIG. 9 is a perspective view showing an indwelled state where the needle assembly shown in FIG. 1 is fixed to a patient's skin with medical tapes.
FIG. 10 is a perspective view of the needle assembly shown in FIG. 1, showing an extended state where the needle tip of the puncture needle is in a needle removal position.
FIG. 11 is a front view of the needle assembly shown in FIG. 10.
FIG. 12 is a cross sectional view taken along line 12-12 of FIG. 11.
FIG. 13 is a perspective view of a needle assembly as a second practical embodiment of the present invention, showing a contracted state where a needle tip of a puncture needle is in a puncture position.
FIG. 14 is a front view of the needle assembly shown in FIG. 13.
FIG. 15 is a cross sectional view taken along line 15-15 of FIG. 14.
FIG. 16 is a perspective view of a needle assembly as a third practical embodiment of the present invention, showing a contracted state where a needle tip of a puncture needle is in a puncture position.
FIG. 17 is a front view of the needle assembly shown in FIG. 16.
FIG. 18 is a right side view of the needle assembly shown in FIG. 17.
FIG. 19 is a cross sectional view taken along line 19-19 of FIG. 17.
FIG. 20 is a perspective view of a wing member constituting the needle assembly shown in FIG. 16.
FIG. 21 is a rear view of the wing member shown in FIG. 20.
FIG. 22 is an arrow view 22 of FIG. 21.
FIG. 23 is a front view showing an embodiment of the needle assembly shown in FIG. 16 during puncture.
FIG. 24 is a perspective view of the needle assembly shown in FIG. 16, showing an extended state where the needle tip of the puncture needle is in a needle removal position.
FIG. 25 is a front view of the needle assembly shown in FIG. 24.
FIG. 26 is a cross sectional view taken along line 26-26 of FIG. 25.
FIG. 27 is a cross sectional view taken along line 27-27 of FIG. 26.
FIG. 28 is a cross sectional view of a lock mechanism of a needle assembly as another practical embodiment of the present invention.
FIG. 29 is a cross sectional view of a lock mechanism of a needle assembly as yet another practical embodiment of the present invention.

### EMBODIMENTS FOR CARRYING OUT THE INVENTION

Practical embodiments of the present invention will be described below in reference to the drawings.

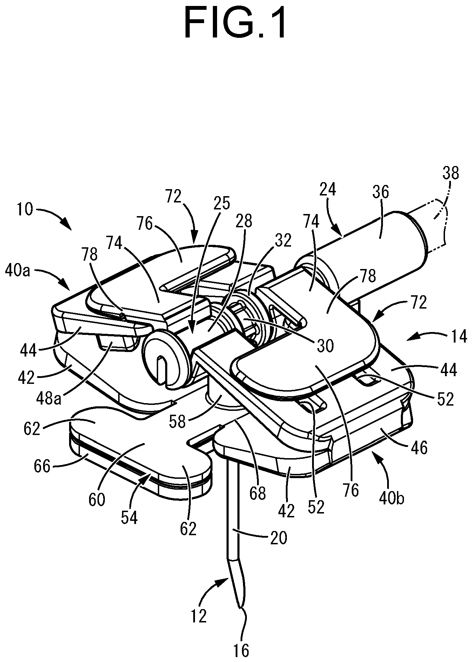

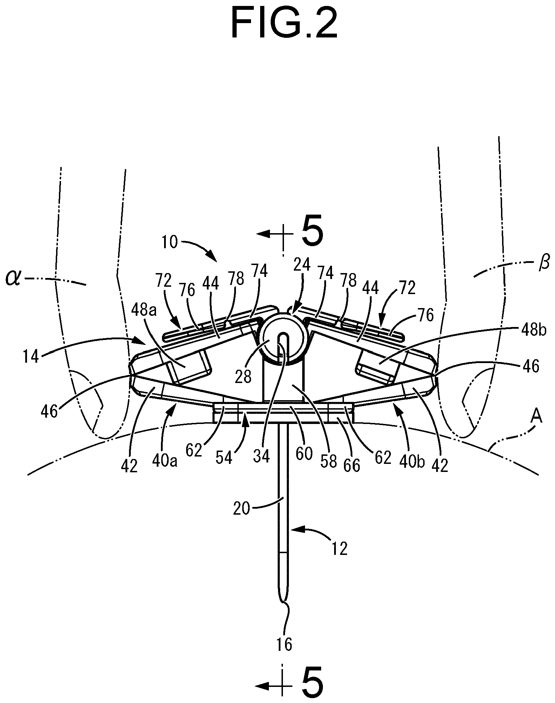

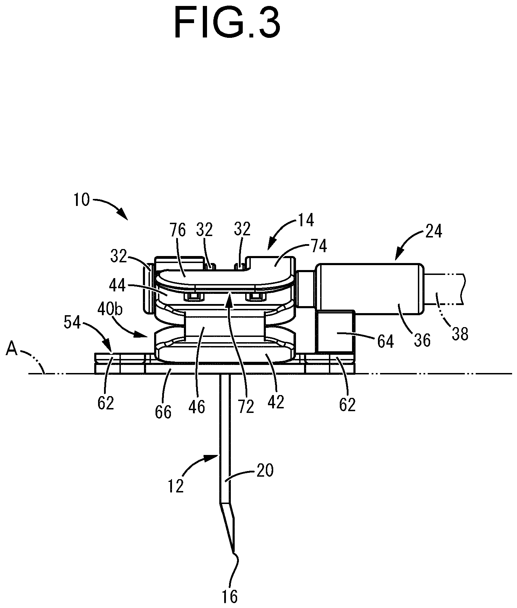

FIGS. 1 to 5 show a needle assembly 10 as a first practical embodiment of the invention in a contracted state. The needle assembly 10 has a structure in which a puncture needle 12 is attached to a wing member 14. In the following description, as a general rule, the vertical direction refers to the vertical direction in FIG. 2, which is the puncture direction and the needle removal direction, the left-right direction refers to the left-right direction in FIG. 2, and the front-back direction refers to the left-right direction in FIG. 3. Note that each direction defined here is set for convenience, and, for example, the vertical direction does not necessarily match the vertical direction when the needle assembly 10 is used.

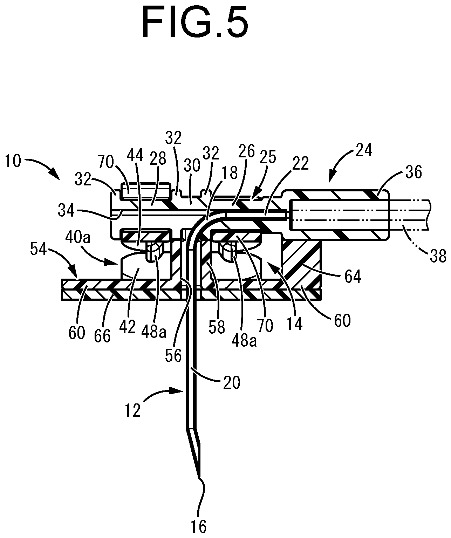

The puncture needle 12 is a hollow metal needle and has a sharp needle tip 16 at the distal end. The puncture needle 12 in this practical embodiment is a Huber needle that punctures a septum of a subcutaneous implantable port, which is not shown. That is, in the puncture needle 12, the needle-tip-16-side end of the lumen opens backward (rightward in FIG. 5) orthogonal to the puncture direction, preventing coring (hollowing of the septum by the puncture needle 12) when the needle punctures the septum. However, the puncture needle may be a hollow needle that opens in the puncture direction, such as an ordinary injection needle.

The puncture needle 12 is bent and extended in an L-shape overall, and has a curved part 18 that curves into a quarter arc (arc with a central angle of 90°). In the puncture needle 12, the distal end side with respect to the curved part 18 constitutes a distal part 20 that extends in the vertical direction, and the proximal end side with respect to the curved part 18 constitutes a proximal part 22 that extends in the front-back direction, which is approximately orthogonal to the puncture direction.

A needle hub 24 is fastened to the proximal part 22 of the puncture needle 12. The needle hub 24 is formed of a rigid resin or the like and has an approximately cylindrical shape. The needle hub 24 includes a coupling part 25 extending in the direction intersecting the puncture direction of the puncture needle 12. The coupling part 25 integrally includes a first coupling part 26 fastened to the proximal part 22 of the puncture needle 12, a second coupling part 28 projecting forward with respect to the puncture needle 12, and a connecting part 30 connecting the first coupling part 26 and the second coupling part 28.

The first coupling part 26 has an approximately cylindrical shape, and the proximal part 22 of the puncture needle 12 is inserted and fastened to the center hole thereof. The outer circumferential surface of the first coupling part 26 includes a flange-shaped part 32 projecting radially outward at the front end and extending in the circumferential direction. The outer circumferential surface of the first coupling part 26 has a smaller diameter behind the flange-shaped part 32.

The connecting part 30 is provided continuously in front of the first coupling part 26, and has an approximately cylindrical shape with a shorter axial length than the first coupling part 26. The curved part 18 of the puncture needle 12 extending forward with respect to the first coupling part 26 projects downward through the lower part of the connecting part 30. The distal part 20 of the puncture needle 12 extends downward below the connecting part 30, and the needle tip 16 is provided at the lower end of the distal part 20. In short, the needle tip 16 of the puncture needle 12 is located below the connecting part 30 provided in the middle of the coupling part 25, and between the first coupling part 26 and the second coupling part 28 in the front-back direction.

The second coupling part 28 is provided continuously in front of the connecting part 30, and is approximately cylindrical like the first coupling part 26. The axially opposite ends of the second coupling part 28 are provided with respective flange-shaped parts 32 similar to that of the first coupling part 26, and the outer circumferential surface of the second coupling part 28 has a smaller diameter axially between the flange-shaped parts 32, 32.

The second coupling part 28 includes a slit 34. The slit 34 penetrates radially from the diametrical center of the second coupling part 28 downward, which is the direction of projection of the distal part 20 of the puncture needle 12, and extends continuously across the entire length in the front-back axial direction. The slit 34 reaches the connecting part 30, while the upper end of the slit 34 is continuous with the central hole of the first coupling part 26 into which the proximal part 22 of the puncture needle 12 is inserted, and the curved part 18 of the puncture needle 12 extends within the slit 34. Thus, for example, the puncture needle 12 can be inserted into the slit 34 from the front against the needle hub 24 formed separately from the puncture needle 12, and the proximal part 22 of the puncture needle 12 can be inserted into the center hole of the first coupling part 26. Then, the proximal part 22 and the first coupling part 26 can be fastened by means of welding, bonding, etc. However, for example, the puncture needle 12 may be inserted when the needle hub 24 is molded so that the needle hub 24 is formed while being fastened to the proximal part 22 of the puncture needle 12.

The needle hub 24 integrally includes a tube connecting part 36 behind the first coupling part 26. The tube connecting part 36 has larger inner and outer diameter dimensions than the first coupling part 26, and one end of a tube 38 is inserted and fastened to the tube connecting part 36. The other end of the tube 38 is connected to a medicine bag, an infusion bag, etc. (not shown), via a connector or another tube, for example, and the medicine or infusion solution is supplied through the tube 38 into the lumen of the puncture needle 12. A clamp can also be provided in the middle of the tube 38 to switch between communicating and blocking the lumen of the tube 38. In the figure, the tube 38 is shown hypothetically by the chain double-dashed line.

The needle hub 24 is attached to the wing member 14. The wing member 14 is made of rigid resin, but by linear-shaped thin-walled portions being partially provided, the wing member 14 has toughness that permits folding (bending) at the thin-walled portions. The wing member 14 includes a pair of variable plate-shaped parts 40a, 40b as shown in FIGS. 1 to 3.

Such a pair of variable plate-shaped parts 40a, 40b are arranged in opposition to each other with one surface of the variable plate-shaped part 40a opposed to one surface of the variable plate-shaped part 40b, and are mutually bendable at the upper portions and the lower portions. Furthermore, the variable plate-shaped parts 40a, 40b each include a first bent part 46 serving as a bent part that is allowed to undergo bending deformation in the middle portion corresponding to each other in the height direction. The variable plate-shaped parts 40a, 40b are configured such that by the bending deformation at both the upper and lower portions and the bent parts in the middle portion in the height direction, the respective middle portions of the variable plate-shaped parts 40a, 40b are close to or remote from each other, whereby deformation in an approximately pantograph-like manner is allowed so as to extend and contract in the vertical direction.

Described more specifically, the variable plate-shaped part 40a/40b has a structure in which a first plate 42 and a second plate 44 are connected by the first bent part 46 serving as an input bent part that is bendable. The first plate 42 and the second plate 44 are gradually thickened toward the first bent part 46, and the area of each end face of the first plate 42 and the second plate 44 on the first bent part 46 side is increased. The first bent part 46 is a thin-walled portion extending linearly in the front-back direction, which is the length direction, between the first plate 42 and the second plate 44. The first bent part 46 is integrally formed with the first plate 42 and the second plate 44, and is allowed to undergo bending deformation due to being thin-walled. In this practical embodiment, the first bent part 46 deforms in a hinge-like manner so as to be bent around a bending axis located on the first bent part 46. However, for example, it would also be acceptable to adopt an embodiment in which the first bent part 46 undergoes bending deformation by being bent in a curved manner around a bending axis that is off the variable plate-shaped part 40a/40b in the plate thickness direction.

The second plate 44 of one variable plate-shaped part 40a includes a pair of engaging parts 48a, 48a, and the second plate 44 of the other variable plate-shaped part 40b includes a pair of engaging parts 48b, 48b. The engaging parts 48a, 48a project in the thickness direction of the second plate 44 and are provided in opposition to each other in the front-back direction, which is the width direction of the second plate 44, and have a structure in which respective claws are provided at the projecting distal end portions so as to project inward in the direction of opposition. The engaging parts 48b, 48b project in the thickness direction of the second plate 44 and are provided in opposition to each other in the width direction of the second plate 44, and have a structure in which respective claws are provided at the projecting distal end portions so as to project outward in the direction of opposition. The distance between the opposed engaging parts 48a, 48a is larger than the distance between the opposed engaging parts 48b, 48b, so that the engaging parts 48b, 48b can be inserted between the opposed engaging parts 48a, 48a. The engaging parts 48a, 48a and the engaging parts 48b, 48b are provided so as to project from the opposed inside faces of the pair of variable plate-shaped parts 40a, 40b, which are arranged in opposition to each other. The second plate 44 includes a pair of through holes 52, 52, which are the marks of a mold for molding the engaging parts 48a, 48a or 48b, 48b. The engaging parts 48a, 48a are provided at the front-back outer opening edges of the through holes 52, 52 in one second plate 44, while the engaging parts 48b, 48b are provided at the front-back inner opening edges of the through holes 52, 52 on the other second plate 44.

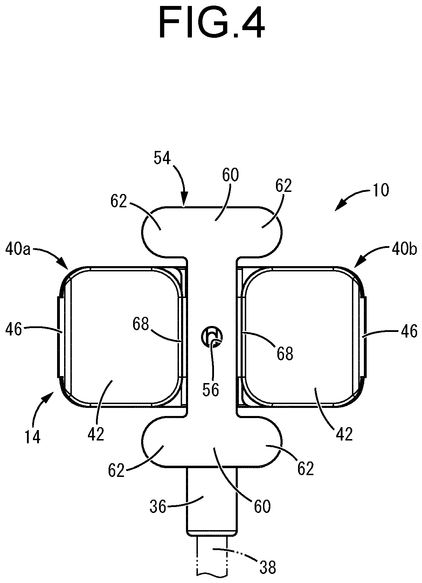

The respective first plates 42 of the variable plate-shaped parts 40a, 40b are connected to a bottom plate 54 at the ends (lower ends) opposite the first bent parts 46 serving as the input bent parts, as shown in FIGS. 1 and 4. The bottom plate 54 has an approximately flat plate shape and has a lower surface that extends roughly orthogonally to the vertical direction including the puncture direction. The bottom plate 54 is vertically penetrated by a needle passage hole 56 in the center portion, through which the puncture needle 12 can be inserted. The bottom plate 54 can be uneven or curved, for example, in consideration of unevenness or curvature of the patient's body surface at the site to be punctured by the needle assembly 10.

In the center portion of the bottom plate 54, a protective tubular part 58 serving as a protective part projects from the upper surface. The protective tubular part 58 is cylindrical and projects upward around the needle passage hole 56 to allow insertion of the puncture needle 12. The upper surface of the protective tubular part 58 curves in a concave shape corresponding to the outer circumferential surface of the tube connecting part 36 of the needle hub 24.

Regarding the bottom plate 54, the front-back opposite end portions constitute projecting parts 60, 60 projecting outward with respect to the variable plate-shaped parts 40a, 40b in the length direction (front-back direction) parallel to the bending axis. The projecting part 60 includes widened parts 62, 62 that project further to the opposite sides in the left-right direction, which is the width direction, and is widened in the left-right direction in comparison with the front-back center portion of the bottom plate 54 connected to the variable plate-shaped parts 40a, 40b. The projecting distal end of the widened parts 62, 62 has an approximately semicircular arc shape with no corners. The back projecting part 60 includes a support part 64 projecting from the upper surface. The support part 64 projects upward in the left-right center of the projecting part 60, and has an approximately rectangular block shape. The support part 64 is located below the tube connecting part 36 of the needle hub 24 and projects toward the tube connecting part 36. The upper surface of the support part 64, which is the projecting distal end face, is a concave curved surface corresponding to the outer circumferential surface of the tube connecting part 36.

A sheet-shaped buffer body 66 having an outer shape that approximately corresponds to the bottom plate 54 is overlapped with the lower surface of the bottom plate 54. The buffer body 66 is made of a resin elastomer or rubber elastic body, etc., and is more flexible than the bottom plate 54 and easily deforms elastically, thereby exerting a buffer effect upon contact. The buffer body 66 is preferably fastened to the lower surface of the bottom plate 54. The needle passage hole 56 of the bottom plate 54 penetrates the buffer body 66. The buffer body 66 is not essential and can be omitted. For example, the buffer body can also be provided on the second plate 44 so that the patient is unlikely to feel pain or other discomfort when the second plate 44 touches a patient's skin A. In FIG. 4, the buffer body 66 is omitted to show the shape of the bottom plate 54.

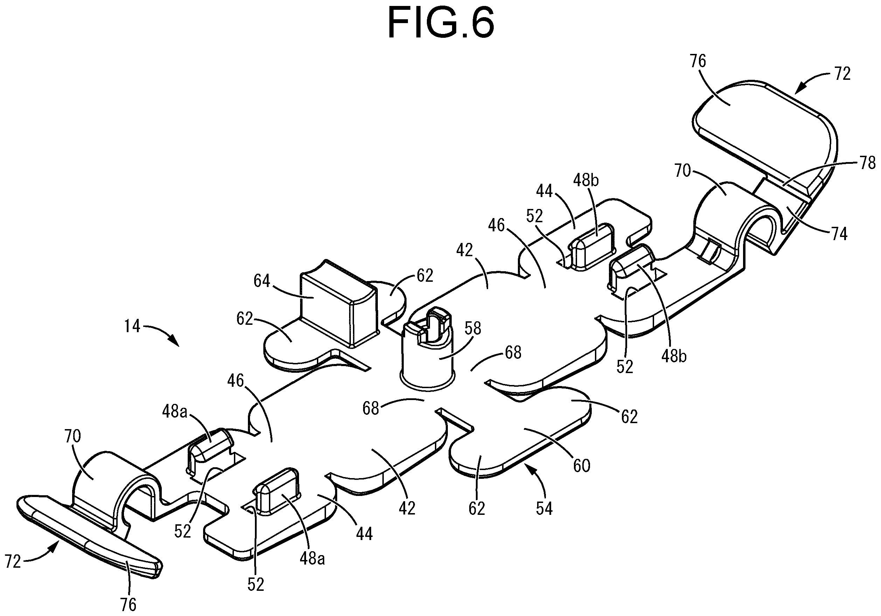

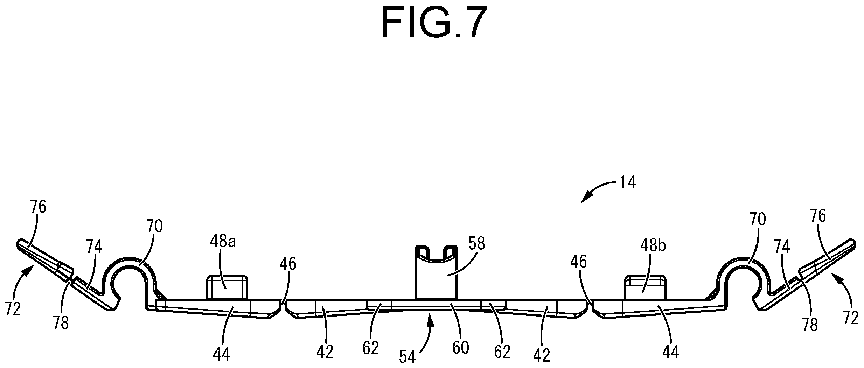

The first plate 42 of the variable plate-shaped part 40a/40b is continuous with the bottom plate 54 in a bendable manner, and the connected portion of the first plate 42 and the bottom plate 54 constitute a second bent part 68 serving as the lower portion. Like the first bent part 46, the second bent part 68 is a thin-walled portion extending linearly in the front-back direction. The second bent part 68 is integrally formed with the first plate 42 and the bottom plate 54, and is allowed to undergo bending deformation due to being thin-walled. The pair of variable plate-shaped parts 40a, 40b are provided on the left and right opposite sides of the bottom plate 54, and the variable plate-shaped parts 40a, 40b are continuously provided on the respective left and right sides of the bottom plate 54. Accordingly, the variable plate-shaped parts 40a, 40b are interconnected at the lower ends via the bottom plate 54, and as shown in FIGS. 6 and 7, the wing member 14 integrally includes the variable plate-shaped parts 40a, 40b and the bottom plate 54.

As shown in FIGS. 1 and 2, the variable plate-shaped parts 40a/40b is coupled at one end to the needle hub 24. That is, the end opposite to the first plate 42 in the second plate 44 (namely, the upper portion) of the variable plate-shaped part 40a/40b includes a fitting groove 70 serving as a fitting part as shown in FIGS. 6 and 7. The fitting groove 70 extends in the front-back direction with a major-arcuate, C-shaped annular cross section that exceeds half a circumference. The fitting groove 70 is shorter than half the length of the second plate 44 in the front-back direction, and its deformation rigidity is lower than that of the second plate 44, allowing the opening portion to expand through elastic flexural deformation. The fitting groove 70 is provided so as to shift backward in one variable plate-shaped part 40a and is provided so as to shift forward in the other variable plate-shaped part 40b.

The first coupling part 26 of the needle hub 24 is fitted into the fitting groove 70 of one variable plate-shaped part 40a, and the second coupling part 28 of the needle hub 24 is fitted into the fitting groove 70 of the other variable plate-shaped part 40b, so that one end of each of the variable plate-shaped parts 40a, 40b is attached to the needle hub 24. The fitting groove 70 of one variable plate-shaped part 40a is attached to the first coupling part 26 behind the distal part 20 of the puncture needle 12, and the fitting groove 70 of the other variable plate-shaped part 40b is attached to the second coupling part 28 in front of the distal part 20 of the puncture needle 12. In short, the pair of variable plate-shaped parts 40a, 40b are attached to the coupling part 25 of the needle hub 24 on the opposite sides of the connecting part 30 in the front-back direction, the connecting part 30 being a portion of the needle hub 24 from which the puncture needle 12 projects. The needle hub 24 is circumferentially rotatable with respect to the fitting grooves 70, 70 of the variable plate-shaped parts 40a, 40b, and the second plates 44, 44 of the variable plate-shaped parts 40a, 40b are coupled to the needle hub 24 with angular change (tilting with the needle hub 24 as the central axis) allowed.

The fitting groove 70 of one variable plate-shaped part 40a is located between the flange-shaped part 32 of the first coupling part 26 and the tube connecting part 36, thereby positioning the variable plate-shaped part 40a and the needle hub 24 in the front-back direction. Similarly, the fitting groove 70 of the other variable plate-shaped part 40b is located between the flange-shaped parts 32, 32 of the second coupling part 28, thereby positioning the variable plate-shaped part 40b and the needle hub 24 in the front-back direction.

The fitting groove 70 is continuous with the second plate 44 at one circumferential end and continuous with a grip piece 72 at the other circumferential end. The grip piece 72 is plate-shaped and is integrally provided with the variable plate-shaped part 40a/40b, and extends from the fitting groove 70, which constitutes the end of the variable plate-shaped part 40a/40b. The grip piece 72 has a length dimension in the front-back direction that is not greater than the length dimension of the variable plate-shaped part 40a/40b in the front-back direction, and is located between the front and back opposite ends of the variable plate-shaped part 40a/40b in the front-back direction without projecting outward in the front-back direction relative to the variable plate-shaped part 40a/40b. The grip piece 72 includes a connection proximal part 74 having the same front-back length dimension as the fitting groove 70, and a grip distal part 76 integrally connected to the distal end of the connection proximal part 74 and having the same front-back length dimension as the second plate 44. The connected portion of the connection proximal part 74 and the grip distal part 76 is thin-walled in the form of a line extending in the width direction, and the thin-walled portion constitutes a movable part 78 that permits bending of the grip distal part 76 with respect to the connection proximal part 74. The connection proximal part 74 may be bendable with respect to the fitting groove 70, but in this practical embodiment, bending with respect to the fitting groove 70 is not allowed. If the connection proximal part 74 is allowed to bend with respect to the fitting groove 70, the movable part can be constituted by the bendable connected portion of the connection proximal part 74 and the fitting groove 70, and the movable part 78 need not be provided in the middle of the grip piece 72.

The wing member 14 with such a structure is formed into a plate shape as shown in FIGS. 6 and 7. By bending the first and second bent parts 46, 68 and fitting the first and second coupling parts 26, 28 of the needle hub 24 into the fitting grooves 70, 70, the wing member 14 is shaped to form the needle assembly 10 in the contracted state shown in FIGS. 1 to 5. Since the wing member 14 is bent after being formed into a plate shape, the engaging parts 48a, 48b and the protective tubular part 58, which are located between the opposed pair of variable plate-shaped parts 40a, 40b in the needle assembly 10, can be easily molded.

In the needle assembly 10 in the contracted state, the distal part 20 of the puncture needle 12 penetrates the needle passage hole 56 of the bottom plate 54. The position of the needle tip 16 is exposed to the outside by projecting downward from the bottom plate 54, so that the position of the needle tip 16 is defined as a puncture position where puncture is possible. In the needle assembly 10 in the contracted state, the vertical approach of the needle hub 24 with respect to the bottom plate 54 is limited by the connecting part 30 of the needle hub 24 coming into contact with the upper surface of the protective tubular part 58. This prevents excessive bending of the first bent part 46, avoiding damage to the pair of variable plate-shaped parts 40a, 40b, and the needle-hub-24-side ends of the variable plate-shaped parts 40a, 40b are held above the first bent part 46.

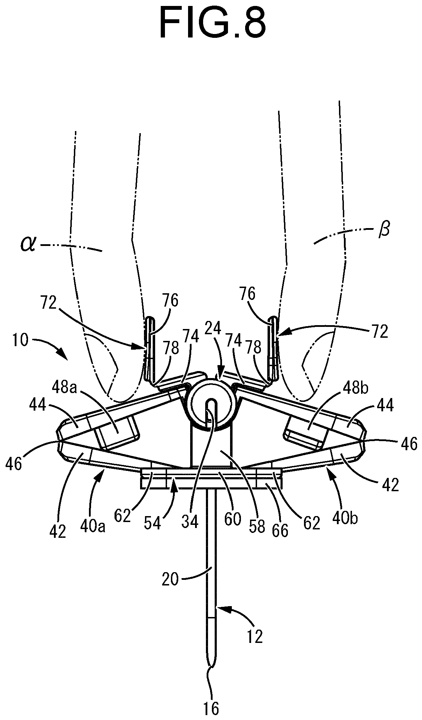

In the needle assembly 10 in the contracted state, as shown in FIG. 8, the grip piece 72 is overlapped on the variable plate-shaped part 40a/40b with the connection proximal part 74 in contact or in close proximity slightly apart, while the grip distal part 76 projects upward away from the variable plate-shaped part 40a/40b by bending of the movable part 78. Then, a health professional grips the grip distal parts 76, 76 of the pair of grip pieces 72, 72 by, for example, pinching them with the index finger a and thumb β of one hand to puncture the patient with the puncture needle 12. Even if the inward force in the left-right direction by gripping and the force in the downward direction, which is the puncture direction, act on the pair of grip distal parts 76, 76, the pair of variable plate-shaped parts 40a, 40b do not undergo extension deformation, and the needle tip 16 of the puncture needle 12 is kept exposed downward.

Regarding the needle assembly 10 stuck into the patient, the buffer body 66 is overlapped in contact with the patient's skin A, and the bottom plate 54 is overlapped in indirect contact with the patient's skin A via the buffer body 66. Accordingly, the punctured needle assembly 10 is in planar contact with the patient's skin A. This reduces pain and discomfort by dispersing the contact pressure on the patient's skin A, and prevents wobbling etc. of the needle assembly 10 by reliably obtaining a large contact area against the patient's skin A. Furthermore, by interposing the flexible buffer body 66 between the bottom plate 54 and the patient's skin A, the contact pressure on the patient's skin A is further reduced, thereby further reducing pain and discomfort.

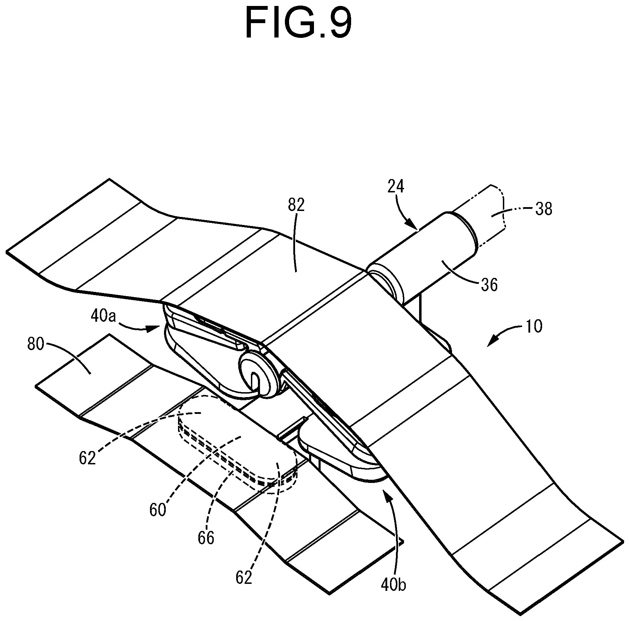

The needle assembly 10 stuck into the patient is fixed to the patient's skin A by medical tapes 80, 82, as shown in FIG. 9. Specifically, the needle assembly 10 is fixed to the patient's skin A by the medical tape 80, which straddles the upper part of the forward projecting part 60 in the left-right direction, and the medical tape 82, which straddles the upper part of the pair of variable plate-shaped parts 40a, 40b in the left-right direction. Thus, in this practical embodiment, the forward projecting part 60 constitutes a fixing piece configured to be fixed to the patient's skin A. Since not only the variable plate-shaped parts 40a, 40b but also the forward projecting part 60 is fixed to the patient's skin A, the needle assembly 10 can be more strongly fixed to the skin A. Besides, since the forward projecting part 60 is overlapped on the patient's skin A, it can be more easily fixed with the medical tape 80 than the second plates 44, 44, etc. of the variable plate-shaped parts 40a, 40b, which are located away from the patient's skin A. Therefore, after puncture of the needle assembly 10, at first, the forward projecting part 60 is easily fixed with the medical tape 80 so that the needle assembly 10 is temporarily positioned, and then the variable plate-shaped parts 40a, 40b are fixed with the medical tape 82, thereby facilitating the fixing work.

When the variable plate-shaped parts 40a, 40b are fixed to the patient's skin A by the medical tape 82, the grip distal parts 76, 76 of the grip pieces 72, 72 are overlapped in contact or in close proximity on the second plates 44, 44 by bending of the movable parts 78, 78. This suppresses projection of the grip distal parts 76,76 so as to reduce the vertical height dimension of the needle assembly 10, while making the upper surface of the needle assembly 10 smooth with few projecting portions. This makes it possible to obtain a large adhesion area of the medical tape 82 to the needle assembly 10, and the needle assembly 10, which is indwelled in a puncture state, is unlikely to be an obstacle.

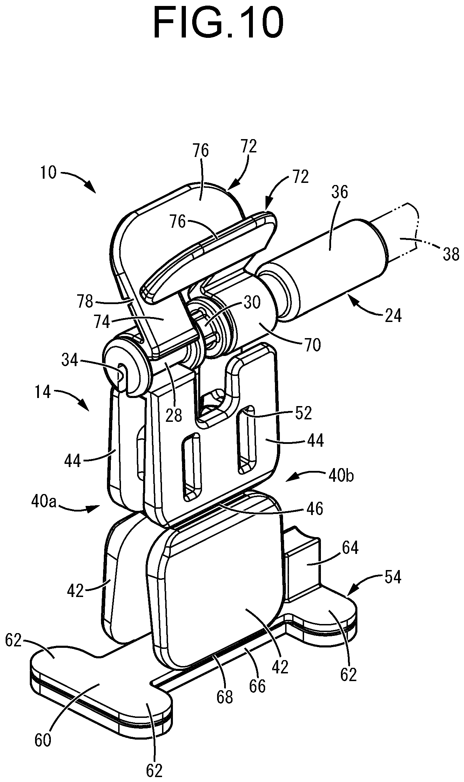

When removing the stuck needle assembly 10 from the patient, first, the medical tapes 80, 82 are peeled off by a health professional, the patient himself/herself, etc. to release fixing of the needle assembly 10 to the patient's skin A. Next, as shown in FIG. 2, the health professional, the patient himself/herself, etc., for example, place the index finger a and thumb β of one hand respectively on the first bent parts 46, 46 of the pair of variable plate-shaped parts 40a, 40b, and apply inward force in the left-right direction, which is the direction of the approach of the first bent parts 46, 46. This causes the first bent parts 46, 46 and the second bent parts 68, 68 to be bent and stretched, while causing the fitting grooves 70, 70 to slide circumferentially with respect to the needle hub 24, so that the variable plate-shaped parts 40a, 40b deforms into an extended state shown in FIGS 10 to 12. In this way, the variable plate-shaped parts 40a, 40b are allowed to bend and stretch at the first bent parts 46, 46 by the bending and stretching of the second bent parts 68, 68 and the circumferential sliding of the fitting grooves 70, 70 with respect to the needle hub 24.

In the contracted state of the pair of variable plate-shaped parts 40a, 40b, the first plate 42 slopes upward from the bottom plate 54 side toward the first bent part 46, while the second plate 44 slopes downward from the fitting groove 70 side toward the first bent part 46. As a result, the variable plate-shaped parts 40a, 40b in the contracted state form an approximately rhombic shape when viewed in the front-back direction. Accordingly, by applying inward force in the left-right direction to the first bent parts 46, 46 of the variable plate-shaped parts 40a, 40b, deformation in the direction in which the slope angle between the first plate 42 and the second plate 44 decreases in each of the variable plate-shaped parts 40a, 40b occurs, and the variable plate-shaped parts 40a, 40b undergo extension deformation.

In this practical embodiment, the first plate 42 and the second plate 44 become thicker toward the first bent part 46, which is the input bent part, and the areas of the end faces of the first plate 42 and the second plate 44 on the first bent part 46 side, which are pressed by the finger α(β), are made large. Accordingly, the contact pressure during pressing the first bent parts 46, 46 by the fingers α, β is reduced, and the pain and discomfort caused by the first bent parts 46, 46 biting into the fingers α, β are reduced, thereby facilitating the operation of inducing extension deformation of the pair of variable plate-shaped parts 40a, 40b.

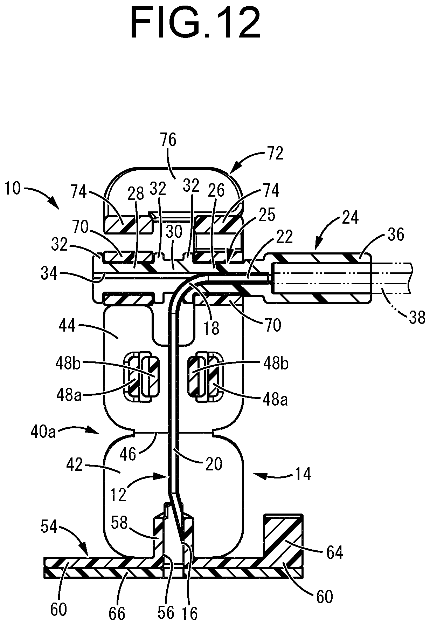

When the pair of variable plate-shaped parts 40a, 40b undergo extension deformation, the needle hub 24 moves in the direction away (upward) from the bottom plate 54 overlapped on the patient's skin A. Accordingly, in the extended state of the variable plate-shaped parts 40a, 40b, the distance from the needle hub 24 to the patient's skin A is longer than the length of the distal part 20 of the puncture needle 12. As a result, as shown in FIG. 12, the needle tip 16 of the puncture needle 12 is located above the lower surface of the bottom plate 54, and the position of the needle tip 16 is defined as a needle removal position where the puncture is released and the needle is removed. In this way, as the needle tip 16 moves away from the patient's skin A, the puncture needle 12 that has punctured the patient is removed from the patient. Then, by separating the bottom plate 54 from the patient's skin A, the needle assembly 10 is removed from the patient.

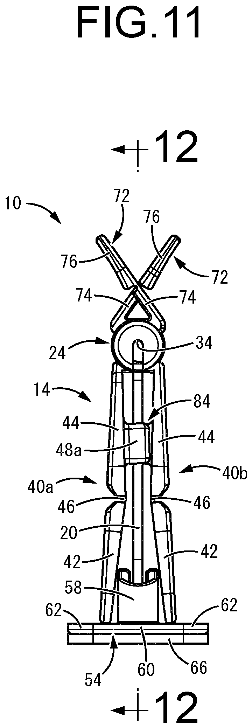

As shown in FIG. 11, the distal part 20 of the removed puncture needle 12 including the needle tip 16 is housed in its entirety between the opposed pair of variable plate-shaped parts 40a, 40b that are in the extended state, and the needle tip 16 is protected without being exposed to the outside. Therefore, it is difficult to accidentally touch the needle tip 16 of the removed puncture needle 12, and excellent safety is realized. Regarding the variable plate-shaped parts 40a, 40b in the extended state, the gap between their opposed faces in the left-right direction is narrowed to such an extent that a finger etc. cannot be inserted. Suitably, the distance between the opposed faces of the variable plate-shaped parts 40a, 40b is set so that when a finger is inserted between the opposed faces of the variable plate-shaped parts 40a, 40b, the fingertip can only enter to the position where it does not touch the puncture needle 12.

As shown in FIG. 12, the needle tip 16 of the puncture needle 12, which is housed between the opposed pair of variable plate-shaped parts 40a, 40b, is housed within the protective tubular part 58 provided in the bottom plate 54. Accordingly, the needle tip 16 of the removed puncture needle 12 is not exposed and accidental puncture of the needle tip 16 is prevented.

Regarding the pair of variable plate-shaped parts 40a, 40b in the extended state, the second plates 44, 44 are opposed to and approach each other in the left-right direction, so that between the engaging parts 48a, 48a provided to the second plate 44 of the one variable plate-shaped part 40a, the engaging parts 48b, 48b provided to the second plate 44 of the other variable plate-shaped part 40b are inserted, and those engaging parts 48a, 48a and 48b, 48b are mutually engaged. This constitutes a lock mechanism 84 that prevents the second plates 44, 44 of the variable plate-shaped parts 40a, 40b from being separated, and the variable plate-shaped parts 40a, 40b are held in the extended state by the lock mechanism 84. Therefore, the variable plate-shaped parts 40a, 40b in the extended state will not undergo contraction deformation, and prevent the puncture needle 12 from projecting downward again, thereby preventing accidental puncture.

When applying force to the first bent parts 46, 46, which are the input bent parts, to induce extension deformation of the pair of the variable plate-shaped parts 40a, 40b, it is assumed that the input positions by the fingers α, β shift or spread toward the second plates 44, 44 due to the extension deformation of the variable plate-shaped parts 40a, 40b. Therefore, the engaging parts 48a, 48a and 48b, 48b are provided on the second plates 44, 44, which is located above the first bent parts 46, 46, which is the opposite side to the puncture direction (the side of the coupling end to the needle hub 24). This makes it easier to apply force to the portions provided with the engaging parts 48a, 48a and 48b, 48b in the extended state of the variable plate-shaped parts 40a, 40b, and to push in the engaging parts 48b, 48b between the opposed engaging parts 48a, 48a to form the lock mechanism 84.

The distal part 20 of the puncture needle 12 extending between the opposed second plates 44, 44 extends between the opposed engaging parts 48a, 48a and between the opposed engaging parts 48b, 48b so as to penetrate the lock mechanism 84 in the vertical direction, and movement of the distal part 20 of the puncture needle 12 in the front-back direction is limited by the lock mechanism 84. This prevents the distal part 20 of the puncture needle 12 from leaving between the opposed pair of variable plate-shaped parts 40a, 40b to project forward or backward, thereby preventing exposure of the needle tip 16 to the outside.

The engaging parts 48a, 48a and 48b, 48b, which constitute the lock mechanism 84, are provided on opposed inner surfaces of the variable plate-shaped parts 40a, 40b, making it difficult to touch them with a finger etc., especially in the extended state of the variable plate-shaped parts 40a, 40b. Therefore, the user is unlikely to release the lock mechanism 84 by accidentally touching the lock mechanism 84, whereby keeping of the extended state of the variable plate-shaped parts 40a, 40b due to the lock mechanism 84, protecting the needle tip 16, etc. can be stably maintained.

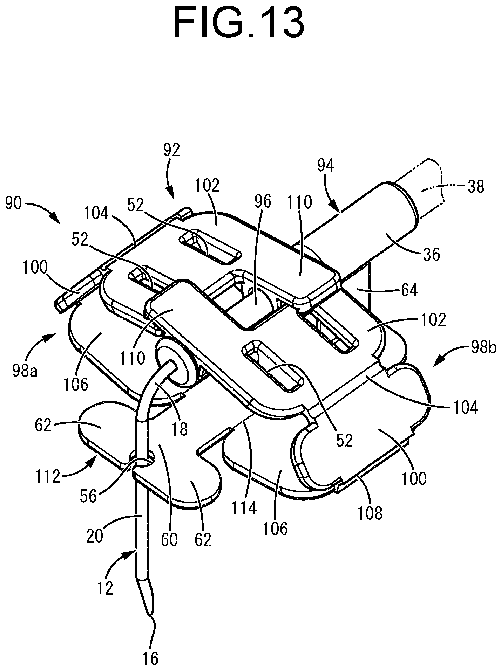

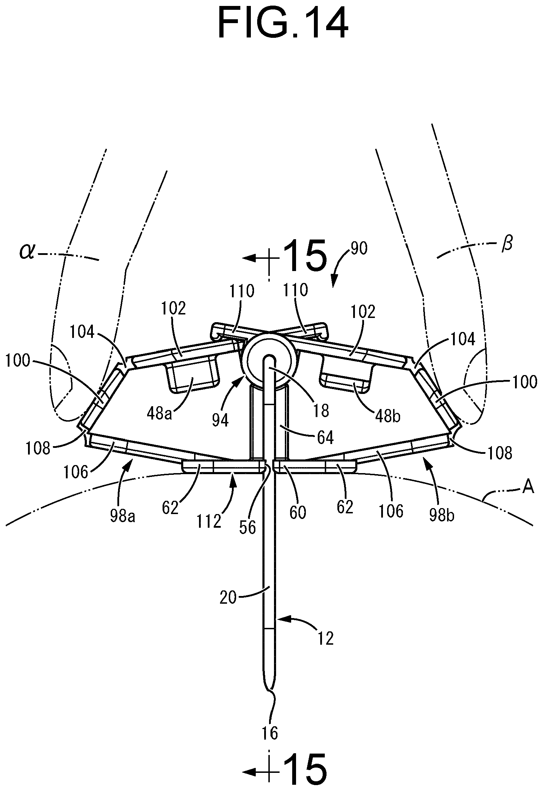

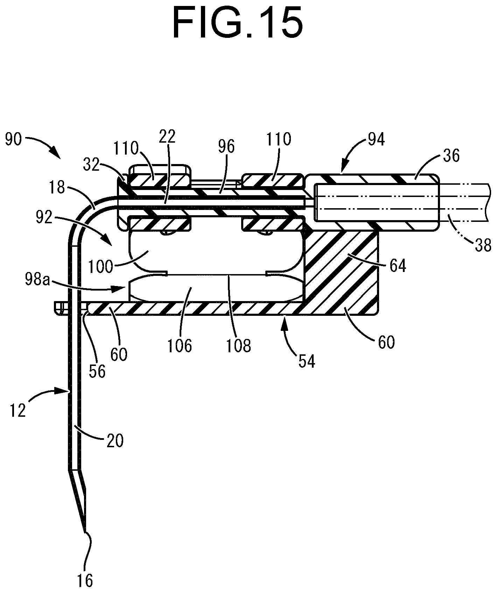

FIGS. 13 to 15 show a needle assembly 90 as a second practical embodiment of the present invention. The needle assembly 90 has a structure in which the puncture needle 12 is attached to a wing member 92. In the following description, components and parts substantially identical with those in the first practical embodiment will be assigned like symbols and not described in any detail.

A needle hub 94 is fastened to the proximal part 22 of the puncture needle 12. The needle hub 94 of this practical embodiment includes a coupling part 96 to which the proximal part 22 of the puncture needle 12 is fastened in an inserted state, and the tube connecting part 36 integrally formed behind the coupling part 96. The coupling part 96 has an approximately cylindrical shape with a smaller diameter than the tube connecting part 36, and includes the flange-shaped part 32 projecting radially outward at the front end.

The wing member 92 includes a pair of variable plate-shaped parts 98a, 98b. The variable plate-shaped part 98a/98b includes an input plate 100 having an approximately flat-plate shape, and has a structure in which a hub coupling plate 102 is continuously provided on one side of the input plate 100 in a bendable manner by a first bent part 104, and a bottom coupling plate 106 is continuously provided on the other side of the input plate 100 in a bendable manner by a second bent part 108. In other words, the variable plate-shaped part 98a/98b includes two bent parts 104, 108, and includes the input plate 100 between those bent parts 104, 108, as well as the hub coupling plate 102 and the bottom coupling plate 106 on the outside of the bent parts 104, 108. In FIGS. 13 to 15, the variable plate-shaped parts 98a, 98b are in a contracted state, and the needle tip 16 of the puncture needle 12 is in a puncture position where puncture is possible.

The hub coupling plate 102 has an approximately flat-plate shape, and slopes downward toward the first bent part 104, which is the connected end with the input plate 100, in the contracted state of the pair of variable plate-shaped parts 98a, 98b. A fitting part 110 with a reduced width dimension is provided at the end of the hub coupling plate 102 opposite to the input plate 100. The fitting part 110 has a C-shaped annular groove that can be fit to the coupling part 96 of the needle hub 94. By fitting the coupling part 96 of the needle hub 94 into the groove of the fitting part 110, the needle hub 94 is held by one end of each of the variable plate-shaped parts 98a, 98b, and the variable plate-shaped parts 98a, 98b are rotatable around the needle hub 94. The fitting part 110 is provided at each of the front end of one variable plate-shaped part 98a and the back end of the other variable plate-shaped part 98b, and those fitting parts 110 are attached to the coupling part 96 of the needle hub 94 at positions separated in the front-back direction. In this practical embodiment, the engaging parts 48a, 48a and 48b, 48b, which constitute the locking mechanism, are provided on the hub coupling plates 102, 102.

The bottom coupling plate 106 has an approximately flat-plate shape, and slopes upward toward the second bent part 108, which is the connected end with the input plate 100, in the contracted state of the pair of variable plate-shaped parts 98a, 98b. At the end of the bottom coupling plate 106 opposite to the input plate 100, a bottom plate 112 is continuously provided in a bendable manner by a third bent part 114. The bottom plate 112 of this practical embodiment has the needle passage hole 56 through which the puncture needle 12 is inserted at the front end of the forward projecting part 60. The needle passage hole 56 vertically penetrates the forward projecting part 60, and opens forward with a width dimension smaller than the outer diameter of the puncture needle 12. The forward projecting part 60 of this practical embodiment is penetrated by the distal part 20 of the puncture needle 12 when the needle assembly 90 is in a contracted state at the time of puncture, and fixation to the patient's skin A with the medical tape is more difficult than in the first practical embodiment, so that function as a fixing piece is not essential. A protective tubular part projecting upward from the periphery of the needle passage hole 56 may be provided to house the needle tip 16 of the removed puncture needle 12.

In this practical embodiment, regarding the puncture needle 12, the curved part 18 and the distal part 20 projecting from the needle hub 94 project further forward than the pair of variable plate-shaped parts 98a, 98b, and the needle tip 16 is positioned further forward than the variable plate-shaped parts 98a, 98b. Since the needle hub 94 is subjected to forces exerted during puncture and removal of the puncture needle 12, it is desirable that the distal part 20 project in the middle of the needle hub 94 to reduce the moment acting on the distal part 20 including the needle tip 16. However, as in this practical embodiment, the distal part 20 may project from the end of the needle hub 94.

The needle assembly 90 with such a structure deforms from the contracted state to the extended state by pinching the pair of input plates 100, 100 with fingers α, β and pressing them inwardly in the left-right direction. Specifically, when applying force to the input plates 100, 100, the first, second, and third bent parts 104, 108, 114 undergo bending and stretching deformation, and one end and the other end of the pair of variable plate-shaped parts 98a, 98b are separated from each other in the vertical direction, which is the direction of extension of the distal part 20 of the puncture needle 12. Accordingly, the needle tip 16 of the puncture needle 12 moves to a non-puncturable needle removal position above the lower surface of the bottom plate 112 overlapped on the patient's skin A, and the puncture needle 12 that has punctured the patient is pulled out.

In this practical embodiment of the needle assembly 90, since the input portion by the fingers α, β is constituted by the plate-shaped input plates 100, 100, the fingers α, β come into planar contact during input and the contact pressure acting on the fingers α, β is dispersed. Therefore, pain, discomfort and the like caused by biting into the fingers α, β are reduced, thereby facilitating pressing operations with the fingers α, β. In this practical embodiment, the first bent part 104 is located more inward in the left-right direction than the second bent part 108, and the input plate 100 located between the first bent part 104 and the second bent part 108 slopes inward in the left-right direction as it goes upward. Thus, the input plates 100, 100 are shaped such that the outer surfaces in the left-right direction are easy to pinch with the fingers from above, and it is easy to pinch the input plates 100, 100 from the side opposite to the patient's skin A (from above) with the fingers in the puncture state and apply force to the needle assembly 90 in the direction of needle removal. This makes the needle removal operation easy.

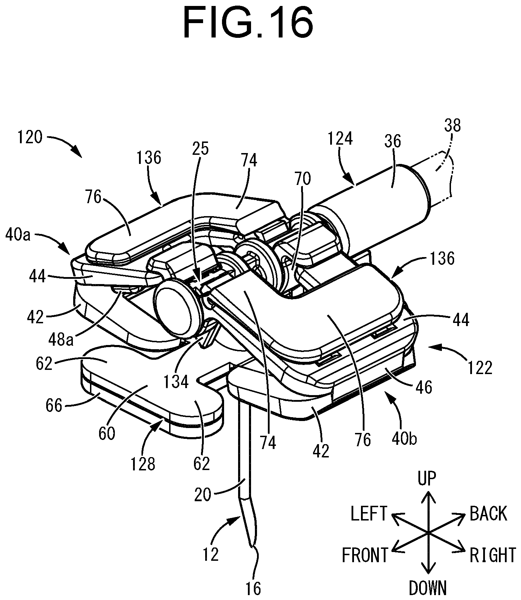

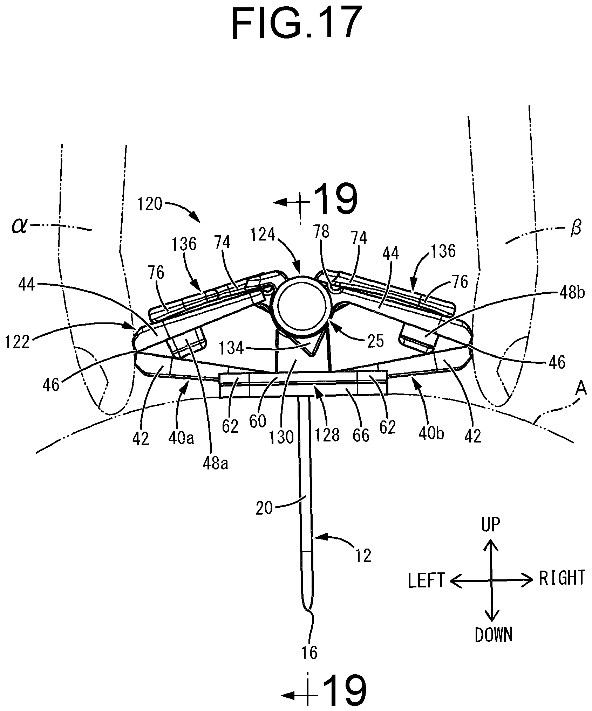

FIGS. 16 to 19 show a needle assembly 120 as a third practical embodiment in a contracted state. The needle assembly 120 has a structure in which the puncture needle 12 is attached to a wing member 122.

Regarding the puncture needle 12, the upper end portion of the distal part 20, the curved part 18, and the proximal part 22, are all fastened to a needle hub 124. The needle hub 124 integrally includes the coupling part 25 and the tube connecting part 36. The coupling part 25 in this practical embodiment does not have the slit 34 as in the first practical embodiment. The puncture needle 12 is inserted when the needle hub 124 is molded, and is integrally fastened to the needle hub 124 in a partially embedded state that cannot be separated. An insertion part 126 extending downward is integrally formed with the connecting part 30 of the needle hub 124. The insertion part 126 is fastened to the upper end portion of the distal part 20 of the puncture needle 12, and has an approximately cylindrical outer circumferential surface.

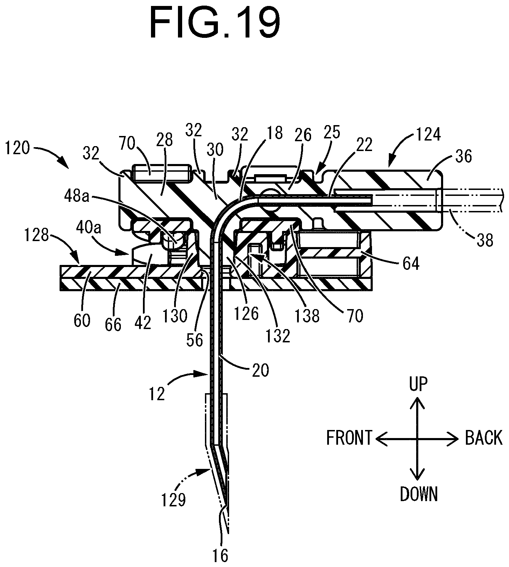

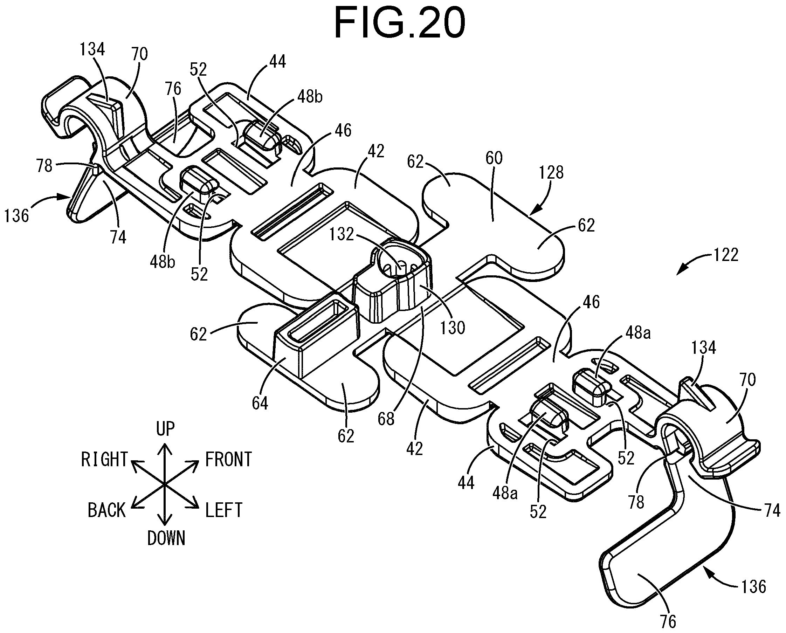

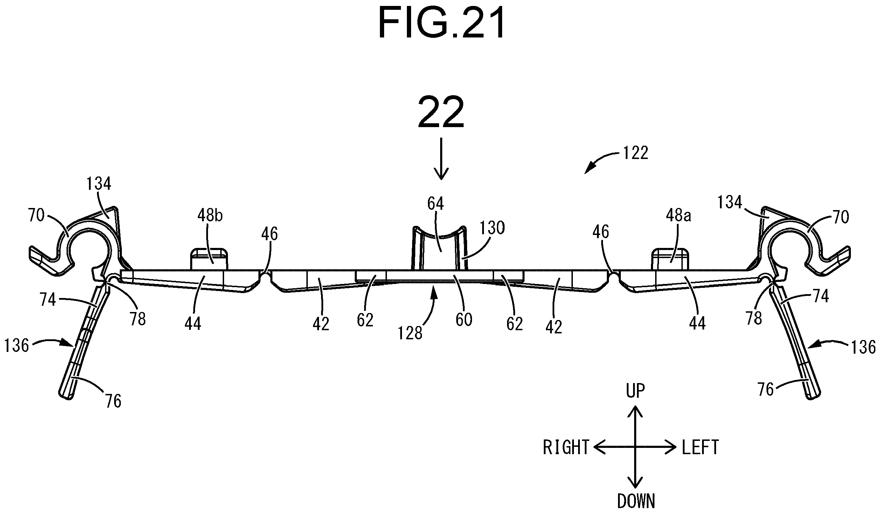

The wing member 122 includes the pair of variable plate-shaped parts 40a, 40b, and a bottom plate 128 that couples the lower ends of the variable plate-shaped parts 40a, 40b to each other. The bottom plate 128 has approximately the same outer shape as the bottom plate 54 of the first practical embodiment, and its center portion is penetrated by the needle passage hole 56 in the vertical direction. The needle passage hole 56 is larger in diameter than the distal part 20 of the puncture needle 12, and is large enough to allow a cap 129 (see FIG. 19) attached to the distal part 20 to pass through. In FIGS. 20 to 22, the wing member 122 is shown in an isolated developed state where it is not attached to the needle hub 124.

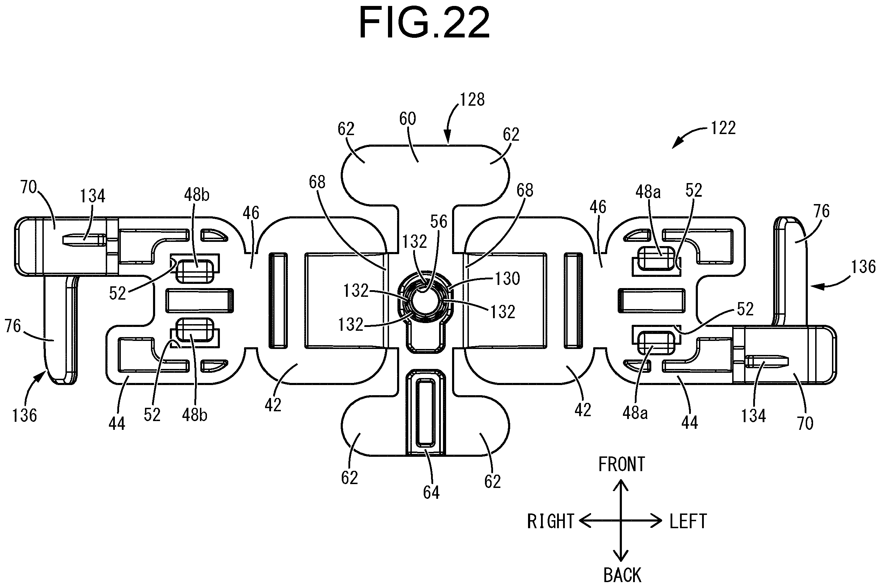

A protective tubular part 130 serving as an external-fit part is provided so as to project upward around the needle passage hole 56 in the bottom plate 128. The protective tubular part 130 has an approximately cylindrical shape, and its rear portion is thick-walled and projects backward. The inner diameter dimension of the protective tubular part 130 is large enough to allow insertion of the insertion part 126 of the needle hub 124. As shown in FIGS. 20 and 22, a plurality of inner projections 132 project radially inward from the inner circumferential surface of the protective tubular part 130. The surface of the inner projection 132 comprises a smoothly continuous curved surface, and in particular, the upper end portion has a tapered shape that slopes radially outward toward the top. The plurality of inner projections 132 are approximately identical to each other and are spaced apart from each other at approximately equal intervals in the circumferential direction of the protective tubular part 130. However, various types of the inner projections having different shapes or sizes may be provided, or the inner projections may be arranged unequally in the circumferential direction.

The wing member 122 of this practical embodiment includes a plurality of lightening recesses opening on the upper surface in the developed state shown in FIGS. 20 to 22 to reduce weight and reduce the amount of forming material while ensuring strength. However, such lightening recesses are not essential, and the wing member 122 may have an approximately flat surface like the wing member 14 of the first practical embodiment.

The upper end of the wing member 122 is attached to the coupling part 25 of the needle hub 124 by the fitting grooves 70, 70 serving as fitting parts. Specifically, the fitting groove 70 provided to one variable plate-shaped part 40a is attached to the outer circumferential surface of the first coupling part 26 of the needle hub 124, while the fitting groove 70 provided to the other variable plate-shaped part 40b is attached to the outer circumferential surface of the second coupling part 28 of the needle hub 124. Accordingly, the needle hub 124 is held by the upper portions of the variable plate-shaped parts 40a, 40b. The fitting grooves 70, 70 are slidable in the circumferential direction with respect to the outer circumferential surface of the needle hub 124, thereby making it possible to change the direction (angle) of extension of the second plates 44, 44 of the wing member 122 from the needle hub 124, and the wing member 122 is allowed to undergo extensional and contractive deformation.

A stopper protrusion 134 protrudes from the outer circumferential surface of the fitting groove 70. The stopper protrusion 134 has a tapered shape that becomes narrower in the circumferential direction of the fitting groove 70 toward the protruding distal end. The outer circumferential surface of the stopper protrusion 134 in this practical embodiment is shaped to be in contact with the fitting groove 70. In this practical embodiment, both of the fitting grooves 70, 70 include the respective stopper protrusions 134, but for example, the stopper protrusion 134 may be provided to only one of the fitting grooves 70, and may be omitted from the other fitting groove 70.

A grip piece 136, which is gripped during puncture, is provided on the upper side of each of the variable plate-shaped parts 40a, 40b. Similar to the grip piece 72 of the first practical embodiment, the grip piece 136 includes the connection proximal part 74 with a narrow width in the front-back direction and the grip distal part 76 with a wide width in the front-back direction, and the connection proximal part 74 projects from near the connected end with the second plate 44 of the fitting groove 70. Regarding the grip piece 136, the movable part 78 is not provided between the connection proximal part 74 and the grip distal part 76, and the grip distal part 76 is not allowed to swing (bend) with respect to the connection proximal part 74. Instead, the movable part 78 is provided at the connected portion between the connection proximal part 74 of the grip piece 136 and the second plate 44, and the entire grip piece 136 is allowed to swing (bend) with respect to the second plate 44.

When attaching the wing member 122 to the needle hub 124, the cap 129 can be mounted on the distal part 20 of the puncture needle 12 in advance, and the cap 129 can be passed from above to below through the needle passage hole 56. By so doing, it is not necessary to mount the cap 129 on the distal part 20 with the needle hub 124 attached to the wing member 122, thereby facilitating manufacture. Then, during puncture, the cap 129 is removed from the puncture needle 12 to expose the needle tip 16 of the distal part 20. In this practical embodiment, in the contracted state of the wing member 122 shown in FIG. 19, the cap 129 is located below the bottom plate 128 and the cap 129 is not inserted into the needle passage hole 56. However, for example, in the contracted state of the wing member 122, the upper end of the cap 129 may be inserted into the needle passage hole 56 so that cap 129 is held by the bottom plate 128. Whereas the cap 129 may be formed of a rigid resin or the like, in this practical embodiment, the cap 129 is formed of a flexible resin tube.

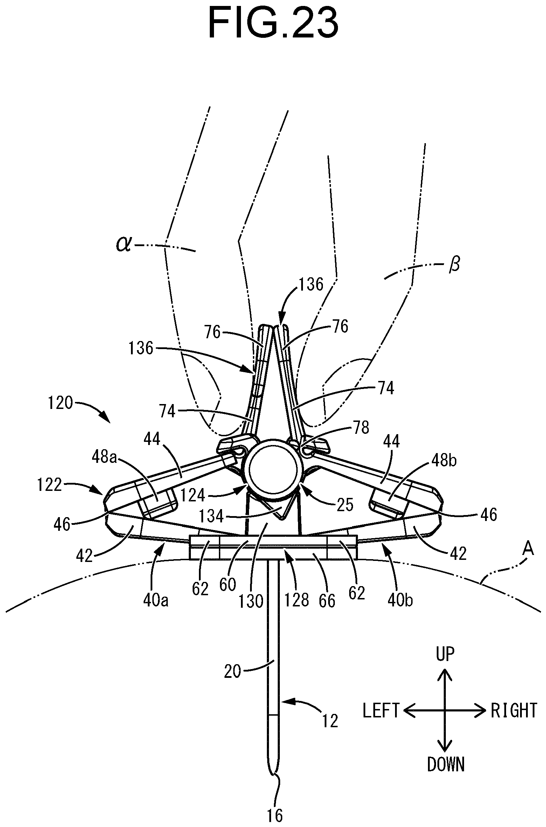

The wing member 122 is in a contracted state during puncture, as shown in FIG. 23, with the distal part 20 of the puncture needle 12 penetrating the needle passage hole 56 of the bottom plate 128 and projecting below the bottom plate 128. With the needle tip 16 of the puncture needle 12 in such a puncture position, as shown in FIG. 19, the insertion part 126 of the needle hub 124 is inserted in the protective tubular part 130 of the bottom plate 128, and the insertion part 126 is fitted in the radial inside of the plurality of inner projections 132 projecting from the inner circumferential surface of the protective tubular part 130. This constitutes a position regulating part 138 that limits the amount of relative displacement between the needle hub 124 and the bottom plate 128 when the needle tip 16 is in the puncture position. The position regulating part 138 of this practical embodiment limits the relative displacement of the needle hub 124 and the bottom plate 128 in the vertical direction and in the direction orthogonal to the vertical direction by the insertion part 126 being fitted into the radial inside of the protective tubular part 130. The position regulating part 138 reduces the displacement (shake) of the puncture needle 12 relative to the wing member 122 in the left-right direction and prevents the puncture needle 12 from accidentally moving upward relative to the bottom plate 128 due to puncture resistance, elasticity of the septum, etc.

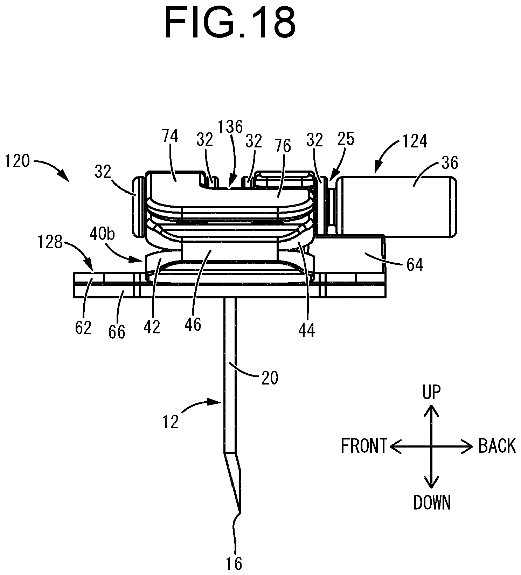

As shown in FIG. 23, the user pinches the grip piece 136 with fingers α, β to puncture the patient with the puncture needle 12. As can be seen in FIG. 18, the distal part 20 of the puncture needle 12 extends in the vertical direction, which is the puncture direction, at a position overlapping with the grip pieces 136, 136 gripped by the user in the front-back direction. In other words, regarding the grip pieces 136, 136 gripped by the user as shown in FIG. 23, the upper-end overlapped portions are located on the extension of the distal part 20 of the puncture needle 12 toward the top. Accordingly, the force is transmitted from the grip pieces 136 to the distal part 20 efficiently and in a balanced manner, which makes the puncture operation easier to perform. When gripped by the user as shown in FIG. 23, the grip pieces 136, 136 left-right symmetrically spread with respect to the extension of the distal part 20 of the puncture needle 12 toward the top. With the puncture needle 12 puncturing the patient, the lower surface of the bottom plate 128 of the wing member 122 is overlapped with the patient's skin A in a state of contact via the buffer body 66.

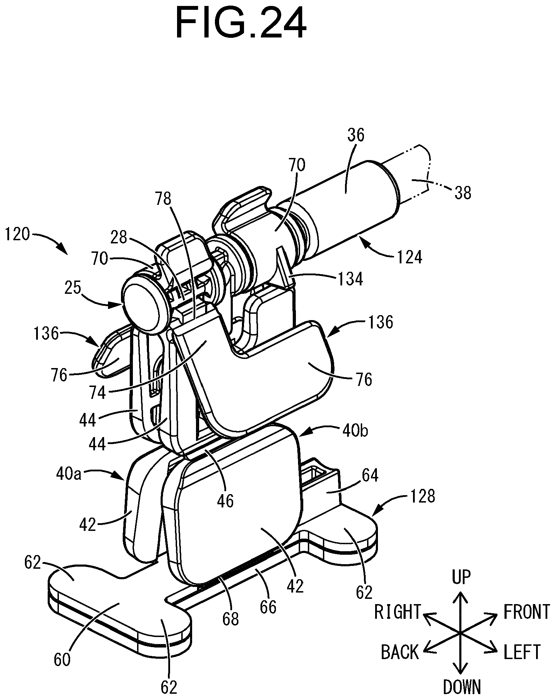

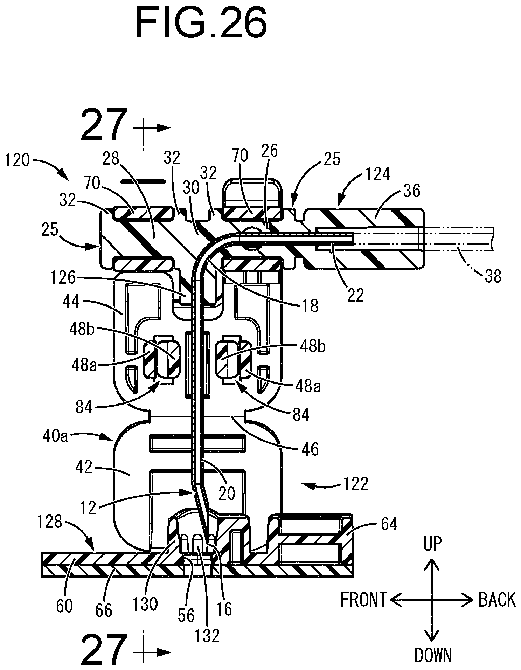

When removing the puncture needle 12 from the patient, the first bent parts 46, 46 of the variable plate-shaped parts 40a, 40b are pushed toward the side of mutual approach in the direction of opposition of the variable plate-shaped parts 40a, 40b, thereby deforming the variable plate-shaped parts 40a, 40b into the extended state shown in FIGS. 24 to 27. Accordingly, the puncture needle 12 moves upward to the needle removal position where the needle tip 16 is above the lower surface of the bottom plate 128. As a result, the puncture needle 12 is removed from the patient, and the needle tip 16 is housed within the protective tubular part 130.

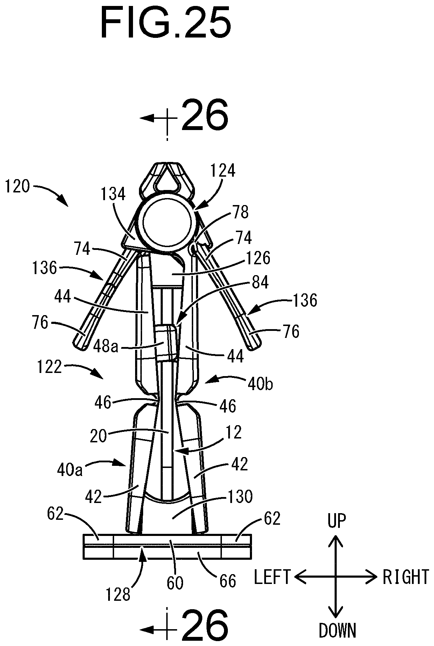

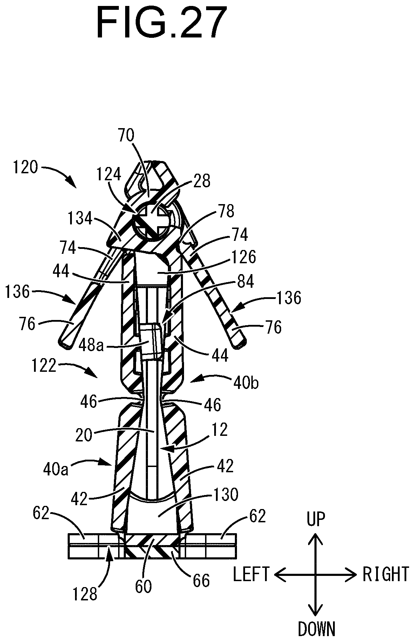

The variable plate-shaped parts 40a, 40b in the extended state are prevented from shifting to the contracted state by the lock mechanism 84 similar to that of the first practical embodiment. Besides, as shown in FIGS. 25 and 27, the shift of the variable plate-shaped parts 40a, 40b to the contracted state with a change in the relative angle of the second plates 44, 44 is also prevented by the stopper protrusions 134, 134, which protrude from the outer circumferential surface of the fitting grooves 70, 70, being engaged to the upper ends of the second plates 44, 44. The shape of the outer circumferential surface of the stopper protrusions 134, 134 are set such that the stopper protrusions 134, 134 are movable over the second plate 44, 44 when the variable plate-shaped parts 40a, 40b in the contracted state shift to the extended state, while the stopper protrusions 134, 134 are engaged without climbing over the second plates 44, 44 when the variable plate-shaped parts 40a, 40b in the extended state shift to the contracted state.

In this way, in the needle assembly 120 of this practical embodiment, the needle tip 16 is configured to be held stably in the puncture position and in the needle removal position, and the situation where the position of the needle tip 16 shifts accidentally from the puncture position to the needle removal position or from the needle removal position to the puncture position is prevented. This makes it possible to avoid troubles such as the puncture needle 12 moving upward by the elasticity of the septum in the puncture state or the needle tip 16 of the puncture needle 12 re-exposing itself below the bottom plate 128 after the needle removal.

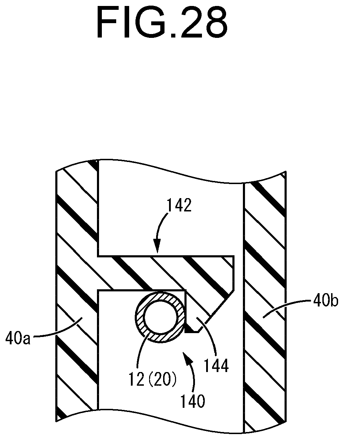

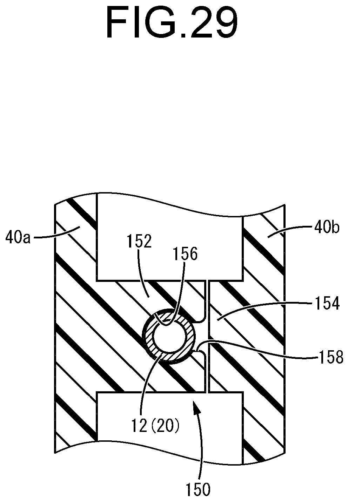

While the present invention has been described in detail hereinabove in terms of the practical embodiments, the invention is not limited by the specific description thereof. For example, the lock mechanisms 84 shown in the preceding practical embodiments are merely exemplary, and are acceptable as long as it is possible to hold the pair of variable plate-shaped parts 40a, 40b in the extended state. For example, only one of the pair of engaging parts (48a, 48b) may be adopted, or other structures such as those illustrated in FIGS. 28 and 29 may be adopted.

Specifically, regarding a lock mechanism 140 shown in FIG. 28, an engaging part 142 projecting from one variable plate-shaped part 40a includes a claw part 144 at its distal end portion. When the pair of variable plate-shaped parts 40a, 40b undergo extension deformation, the puncture needle 12 that has climbed over the claw part 144 is engaged with the claw part 144, thereby providing the lock mechanism 140. In the lock mechanism 140, separation between one variable plate-shaped part 40a and the puncture needle 12 is limited by the claw part 144 being engaged with the puncture needle 12, thereby holding the variable plate-shaped parts 40a, 40b in the extended state.

A lock mechanism 150 shown in FIG. 29 includes a needle holder 152 projecting from one variable plate-shaped part 40a toward the other variable plate-shaped part 40b, and a pushing part 154 projecting from the other variable plate-shaped part 40b toward the one variable plate-shaped part 40a. The needle holder 152 has an approximately rectangular block shape, and includes a needle storage hole 156 penetrating in the vertical direction (direction orthogonal to the paper surface in FIG. 29) and an insertion opening 158 opening the needle storage hole 156 over the entire length toward the projecting distal end side of the needle holder 152. The pushing part 154 has an approximately rectangular block shape, and in the extended state of the pair of variable plate-shaped parts 40a, 40b, the projecting distal end is close to or in contact with the projecting distal end of the needle holder 152. When the variable plate-shaped parts 40a, 40b undergo extension deformation, the projecting distal end face of the pushing part 154 is pressed against the outer circumferential surface of the puncture needle 12, and the puncture needle 12 is pushed into the insertion opening 158 of the needle holder 152 by the pushing part 154, whereby the puncture needle 12 is inserted into the needle storage hole 156 through the insertion opening 158. The width of the insertion opening 158 is smaller than the diameter of the needle storage hole 156, so that the puncture needle 12 inserted into the needle passage hole 156 will not exit from the needle storage hole 156 through the insertion opening 158. Thus, separation between one variable plate-shaped part 40a and the puncture needle 12 is limited, and the lock mechanism 150 holds the variable plate-shaped parts 40a, 40b in the extended state.

The puncture needle 12 is not necessarily limited to the one having a curved part 18 and extending in an L-shape, but may be, for example, a straight hollow needle constituted only by a portion corresponding to the distal part 20. In this case, for example, the pair of variable plate-shaped parts 40a, 40b can be attached to the needle hub as in the preceding first practical embodiment, by making the needle hub T-shaped in cross section to provide a portion projecting toward the puncture direction (downward) in the middle of the needle hub, and fastening the proximal end part of the puncture needle to said projecting portion. Alternatively, the needle hub may be made L-shaped in cross section to have a portion projecting toward the puncture direction (downward) at the distal end portion of the needle hub, and the proximal end part of the puncture needle may be fastened to said projecting portion. This makes it possible to attach the variable plate-shaped parts 40a, 40b to the needle hub behind the needle tip 16 as in the preceding practical embodiment.

When the bottom plate 54 is provided, the bottom plate 54 does not necessarily have to include the projecting parts 60, 60 projecting to front-back opposite sides, but may include only one projecting part 60 projecting to front-back one side, or may include a projecting part projecting in the left-right direction, or need not include a projecting part. Besides, the projecting part 60 need not include the widened part 62, and the entire bottom plate 54 including the projecting part 60 may have an approximately constant width.

The external-fit part of the position regulating part that regulates the relative displacement between the needle hub and the bottom plate is not limited to a tubular shape such as the protective tubular part 130 of the third practical embodiment. For example, the external-fit part comprises a pair of protrusions projecting upward from the bottom plate 128 toward the needle hub 124, and the position regulating part may be constituted by the insertion part 126 of the needle hub 124 being inserted between those protrusions. The position regulating part may alternatively be constituted by insertion of the insertion part 126 into the needle passage hole 56 of the bottom plate 128. The insertion part 126 may be inserted into the protective tubular part 130 serving as the external-fit part with some gap, thereby regulating only the relative displacement between the needle hub 124 and the bottom plate 128 in the horizontal direction. In this case, the inner projection 132 projecting from the inner circumferential surface of the protective tubular part 130 may be omitted.

The vertical position regulation between the needle hub and the bottom plate is not necessarily limited to the one utilizing sliding resistance caused by fitting of the insertion part and the external-fit part. For example, a claw projecting from the outer surface of the insertion part and a claw projecting from the inner surface of the external-fit part can be engaged in the vertical direction, thereby providing the position regulating part in the vertical direction.

The grip pieces 72, 136 shown in the preceding practical embodiments are only examples, and the specific embodiment of the grip piece should not be construed as limited to the preceding practical embodiments. For example, the shape of the grip piece is not limited to the plate shape as in the preceding practical embodiments, but may have a different shape such as a rod shape. The number of the grip pieces is not limited to two, but may be one, for example, or three or more. The grip pieces can be provided on the needle hub 24, for example. The grip pieces can be provided in a pair to the needle hub 24 in a bendable manner, or can be fixedly provided so as to project radially outward from the needle hub 24 in an embodiment that will not be bent.

### KEYS TO SYMBOLS

- 10: needle assembly (first practical embodiment)
- 12: puncture needle
- 14: wing member
- 16: needle tip
- 18: curved part
- 20: distal part
- 22: proximal part
- 24: needle hub
- 25: coupling part
- 26: first coupling part
- 28: second coupling part
- 30: connecting part
- 32: flange-shaped part
- 34: slit
- 36: tube connecting part
- 38: tube
- 40 (40a, 40b): variable plate-shaped part
- 42: first plate
- 44: second plate
- 46: first bent part (input bent part)
- 48 (48a, 48b): engaging part
- 52: through hole
- 54: bottom plate
- 56: needle passage hole
- 58: protective tubular part (protective part)
- 60: projecting part (fixing piece)
- 62: widened part
- 64: support part
- 66: buffer body
- 68: second bent part (lower portion)
- 70: fitting groove (upper portion, fitting part)
- 72: grip piece
- 74: connection proximal part
- 76: grip distal part
- 78: movable part
- 80: medical tape
- 82: medical tape
- 84: lock mechanism
- 90: needle assembly (second practical embodiment)
- 92: wing member
- 94: needle hub
- 96: coupling part
- 98 (98a, 98b): variable plate-shaped part
- 100: input plate
- 102: hub coupling plate
- 104: first bent part
- 106: bottom coupling plate
- 108: second bent part
- 110: fitting part
- 112: bottom plate
- 114: third bent part
- 120: needle assembly (third practical embodiment)
- 122: wing member
- 124: needle hub
- 126: insertion part
- 128: bottom plate
- 129: cap
- 130: protective tubular part (protective part, external-fit part)
- 132: inner projection
- 134: stopper protrusion
- 136: grip piece
- 138: position regulating part
- 140: lock mechanism (another practical embodiment)
- 142: engaging part
- 144: claw part
- 150: lock mechanism (another practical embodiment)
- 152: needle holder
- 154: pushing part
- 156: needle storage hole
- 158: insertion opening
- A: patient's skin
- α: index finger
- β: thumb

## Claims

1. A needle assembly comprising:
a pair of variable plate-shaped parts arranged in opposition to each other and being mutually bendable at both upper portions and lower portions, the pair of variable plate-shaped parts each including a bent part in a middle portion in a height direction, the bent part being allowed to undergo bending deformation; and
a needle hub of a puncture needle held by the upper portions of the pair of variable plate-shaped parts such that the pair of variable plate-shaped parts are allowed to deform so as to extend and contract in a vertical direction that is a puncture direction of the puncture needle, wherein
in a contracted state of the pair of variable plate-shaped parts, a position of a needle tip of the puncture needle is defined as a puncture position where puncture is possible, while in an extended state of the pair of variable plate-shaped parts, a position of the needle tip is defined as a needle removal position where the puncture needle is removed, and
a lock mechanism configured to hold the pair of variable plate-shaped parts in the extended state is provided on opposed inside faces of the pair of variable plate-shaped parts.

2. The needle assembly according to claim 1, wherein the lock mechanism is provided above the bent parts of the variable plate-shaped parts.

3. A needle assembly comprising:
a pair of variable plate-shaped parts arranged in opposition to each other and being mutually bendable at both upper portions and lower portions, the pair of variable plate-shaped parts each including a bent part in a middle portion in a height direction, the bent part being allowed to undergo bending deformation; and
a needle hub of a puncture needle held by the upper portions of the pair of variable plate-shaped parts such that the pair of variable plate-shaped parts are allowed to deform so as to extend and contract in a vertical direction that is a puncture direction of the puncture needle, wherein
in a contracted state of the pair of variable plate-shaped parts, a position of a needle tip of the puncture needle is defined as a puncture position where puncture is possible, while in an extended state of the pair of variable plate-shaped parts, a position of the needle tip is defined as a needle removal position where the puncture needle is removed, and
the lower portions of the pair of variable plate-shaped parts are interconnected via a bottom plate that is configured to be overlapped on a skin of a patient.

4. The needle assembly according to claim 3, wherein the bottom plate includes a projecting part projecting outward with respect to the variable plate-shaped parts in a length direction parallel to a bending axis of the variable plate-shaped parts.

5. The needle assembly according to claim 4, wherein the bottom plate is widened at the projecting part.

6. The needle assembly according to claim 4 or 5, wherein the projecting part is arranged in opposition to the needle hub, and the projecting part includes a support part projecting toward the needle hub.

7. A needle assembly comprising:
a pair of variable plate-shaped parts arranged in opposition to each other and being mutually bendable at both upper portions and lower portions, the pair of variable plate-shaped parts each including a bent part in a middle portion in a height direction, the bent part being allowed to undergo bending deformation; and
a needle hub of a puncture needle held by the upper portions of the pair of variable plate-shaped parts such that the pair of variable plate-shaped parts are allowed to deform so as to extend and contract in a vertical direction that is a puncture direction of the puncture needle, wherein
in a contracted state of the pair of variable plate-shaped parts, a position of a needle tip of the puncture needle is defined as a puncture position where puncture is possible, while in an extended state of the pair of variable plate-shaped parts, a position of the needle tip is defined as a needle removal position where the puncture needle is removed,
the needle hub includes a coupling part extending in a direction intersecting the puncture direction of the puncture needle,
the puncture needle projects in the puncture direction from a lengthwise middle of the coupling part, and
the upper portions of the pair of variable plate-shaped parts are attached to the coupling part on lengthwise opposite sides of a portion from which the puncture needle projects.

8. The needle assembly according to claim 7, wherein
the upper portions of the pair of variable plate-shaped parts comprise respective fitting parts, and
the coupling part is rotatably fitted in the fitting parts of the pair of variable plate-shaped parts such that mutual bending of the pair of variable plate-shaped parts is permitted.

9. A needle assembly comprising:
a pair of variable plate-shaped parts arranged in opposition to each other and being mutually bendable at both upper portions and lower portions, the pair of variable plate-shaped parts each including a bent part in a middle portion in a height direction, the bent part being allowed to undergo bending deformation; and
a needle hub of a puncture needle held by the upper portions of the pair of variable plate-shaped parts such that the pair of variable plate-shaped parts are allowed to deform so as to extend and contract in a vertical direction that is a puncture direction of the puncture needle, wherein
in a contracted state of the pair of variable plate-shaped parts, a position of a needle tip of the puncture needle is defined as a puncture position where puncture is possible, while in an extended state of the pair of variable plate-shaped parts, a position of the needle tip is defined as a needle removal position where the puncture needle is removed,
a grip piece configured to be gripped during puncture of the puncture needle is provided, and
the grip piece is located between opposite ends of the variable plate-shaped parts in a length direction parallel to a bending axis of the variable plate-shaped parts.

10. The needle assembly according to claim 9, wherein
the grip piece extends from at least one of the variable plate-shaped parts, and
a movable part is provided, the movable part permitting bending of the grip piece with respect to the at least one of the variable plate-shaped parts.

11. A needle assembly comprising:
a pair of variable plate-shaped parts arranged in opposition to each other and being mutually bendable at both upper portions and lower portions, the pair of variable plate-shaped parts each including a bent part in a middle portion in a height direction, the bent part being allowed to undergo bending deformation; and
a needle hub of a puncture needle held by the upper portions of the pair of variable plate-shaped parts such that the pair of variable plate-shaped parts are allowed to deform so as to extend and contract in a vertical direction that is a puncture direction of the puncture needle, wherein
in a contracted state of the pair of variable plate-shaped parts, a position of a needle tip of the puncture needle is defined as a puncture position where puncture is possible, while in an extended state of the pair of variable plate-shaped parts, a position of the needle tip is defined as a needle removal position where the puncture needle is removed, and
a protective part is provided, the protective part being configured to prevent the needle tip of the puncture needle from projecting to an outside in the extended state of the pair of variable plate-shaped parts.

12. A needle assembly comprising:
a pair of variable plate-shaped parts arranged in opposition to each other and being mutually bendable at both upper portions and lower portions, the pair of variable plate-shaped parts each including a bent part in a middle portion in a height direction, the bent part being allowed to undergo bending deformation; and
a needle hub of a puncture needle held by the upper portions of the pair of variable plate-shaped parts such that the pair of variable plate-shaped parts are allowed to deform so as to extend and contract in a vertical direction that is a puncture direction of the puncture needle, wherein
in a contracted state of the pair of variable plate-shaped parts, a position of a needle tip of the puncture needle is defined as a puncture position where puncture is possible, while in an extended state of the pair of variable plate-shaped parts, a position of the needle tip is defined as a needle removal position where the puncture needle is removed, and
the bent part of each variable plate-shaped part is defined as an input bent part configured to be pressed when the variable plate-shaped part undergoes extension deformation, and the variable plate-shaped part is thickened toward the input bent part.

13. A needle assembly comprising:
a pair of variable plate-shaped parts arranged in opposition to each other and being mutually bendable at both upper portions and lower portions, the pair of variable plate-shaped parts each including at least one bent part in a middle portion in a height direction, the at least one bent part being allowed to undergo bending deformation; and
a needle hub of a puncture needle held by the upper portions of the pair of variable plate-shaped parts such that the pair of variable plate-shaped parts are allowed to deform so as to extend and contract in a vertical direction that is a puncture direction of the puncture needle, wherein
in a contracted state of the pair of variable plate-shaped parts, a position of a needle tip of the puncture needle is defined as a puncture position where puncture is possible, while in an extended state of the pair of variable plate-shaped parts, a position of the needle tip is defined as a needle removal position where the puncture needle is removed, and
the at least one bent part of each variable plate-shaped part comprises a plurality of bent parts, and a portion between two of the bent parts adjacent to each other in the height direction is defined as an input plate configured to be pressed when the variable plate-shaped part undergoes extension deformation.

14. A needle assembly comprising:
a pair of variable plate-shaped parts arranged in opposition to each other and being mutually bendable at both upper portions and lower portions, the pair of variable plate-shaped parts each including a bent part in a middle portion in a height direction, the bent part being allowed to undergo bending deformation; and
a needle hub of a puncture needle held by the upper portions of the pair of variable plate-shaped parts such that the pair of variable plate-shaped parts are allowed to deform so as to extend and contract in a vertical direction that is a puncture direction of the puncture needle, wherein
in a contracted state of the pair of variable plate-shaped parts, a position of a needle tip of the puncture needle is defined as a puncture position where puncture is possible, while in an extended state of the pair of variable plate-shaped parts, a position of the needle tip is defined as a needle removal position where the puncture needle is removed, and
a fixing piece configured to be overlapped on and fixed to a skin of a patient projects outward with respect to the pair of variable plate-shaped parts in a length direction parallel to a bending axis of the variable plate-shaped parts.

15. The needle assembly according to any one of claims 1-14, wherein an elastically deformable buffer body is provided on a surface configured to be overlapped in contact with a skin of a patient.

16. The needle assembly according to any one of claims 1-15, wherein the needle tip of the puncture needle is configured to be housed between the pair of variable plate-shaped parts in the extended state of the pair of variable plate-shaped parts.

17. A needle assembly comprising:
a pair of variable plate-shaped parts arranged in opposition to each other and being mutually bendable at both upper portions and lower portions, the pair of variable plate-shaped parts each including a bent part in a middle portion in a height direction, the bent part being allowed to undergo bending deformation; and
a needle hub of a puncture needle held by the upper portions of the pair of variable plate-shaped parts such that the pair of variable plate-shaped parts are allowed to deform so as to extend and contract in a vertical direction that is a puncture direction of the puncture needle, wherein
in a contracted state of the pair of variable plate-shaped parts, a position of a needle tip of the puncture needle is defined as a puncture position where puncture is possible, while in an extended state of the pair of variable plate-shaped parts, a position of the needle tip is defined as a needle removal position where the puncture needle is removed,
a grip piece configured to be gripped during puncture of the puncture needle is provided, and
the puncture needle extends in the puncture direction at a position overlapping with the grip piece in a bending axis direction of the variable plate-shaped parts.

18. A needle assembly comprising:
a pair of variable plate-shaped parts arranged in opposition to each other and being mutually bendable at both upper portions and lower portions, the pair of variable plate-shaped parts each including a bent part in a middle portion in a height direction, the bent part being allowed to undergo bending deformation; and
a needle hub of a puncture needle held by the upper portions of the pair of variable plate-shaped parts such that the pair of variable plate-shaped parts are allowed to deform so as to extend and contract in a vertical direction that is a puncture direction of the puncture needle, wherein
in a contracted state of the pair of variable plate-shaped parts, a position of a needle tip of the puncture needle is defined as a puncture position where puncture is possible, while in an extended state of the pair of variable plate-shaped parts, a position of the needle tip is defined as a needle removal position where the puncture needle is removed,
the lower portions of the pair of variable plate-shaped parts are interconnected via a bottom plate that is configured to be overlapped on a skin of a patient, and
a position regulating part is provided, the position regulating part being configured to limit relative displacement between the needle hub and the bottom plate when the needle tip is in the puncture position.

19. The needle assembly according to claim 16, wherein
the bottom plate includes a needle passage hole through which the puncture needle is configured to be inserted when the needle tip of the puncture needle is in the puncture position, and an external-fit part projecting toward the needle hub from around the needle passage hole,
the needle hub includes an insertion part extending toward the bottom plate, and
the position regulating part is constituted by the insertion part being inserted into the external-fit part, the position regulating part limiting the relative displacement between the needle hub and the bottom plate in at least one of a direction of separation of the needle hub and the bottom plate and a direction of opposition of the pair of variable plate-shaped parts.

20. A needle assembly comprising a pair of variable plate-shaped parts arranged in opposition to each other and being mutually bendable at both upper portions and lower portions, the pair of variable plate-shaped parts each including a bent part in a middle portion in a height direction, the bent part being allowed to undergo bending deformation, wherein
the upper portions of the pair of variable plate-shaped parts comprise respective fitting parts of groove shape attached to an outer circumferential surface of a needle hub of a puncture needle, the fitting parts being slidable with respect to the needle hub in a circumferential direction of the needle hub such that the pair of variable plate-shaped parts are allowed to deform so as to extend and contract in a vertical direction that is a puncture direction of the puncture needle,
in a contracted state of the pair of variable plate-shaped parts, a position of a needle tip of the puncture needle is defined as a puncture position where puncture is possible, while in an extended state of the pair of variable plate-shaped parts, a position of the needle tip is defined as a needle removal position where the puncture needle is removed, and
a stopper protrusion is provided on an outer circumferential surface of the fitting part of at least one of the variable plate-shaped parts, the stopper protrusion being configured to be engaged to the other of the variable plate-shaped parts to prevent the needle tip from moving from the needle removal position to the puncture position.
